# EUROPÄISCHE PATENTSCHRIFT

(11) **EP 1 869 138 B1**
(45) Veröffentlichungstag und Bekanntmachung des Hinweises auf die Patenterteilung: **25.11.2009**
(21) Anmeldenummer: 06725390.6
(22) Anmeldetag: 29.03.2006
(51) Int. Cl.: C09K 8/32, C09K 8/035, C09K 8/36

(54) **BOHRSPÜLUNG ENTHALTEND HYDROPHOBIN**
DRILLING FLUID CONTAINING HYDROPHOBIN
LAVAGE DE FORAGE AU MOYEN D'HYDROPHOBINE

(30) Priorität: 01.04.2005 EP 05007208; 04.08.2005 EP 05016962
(43) Veröffentlichungstag der Anmeldung: 26.12.2007
(73) Patentinhaber: BASF SE, 67056 Ludwigshafen (DE)
(72) Erfinder: GUZMANN, Marcus, 69242 Mühlhausen (DE); LIU, Yaqian, 67065 Ludwigshafen (DE); BAUS, Ulf, 69221 Dossenheim (DE)
(74) Vertreter: Jacobi, Markus Alexander
(86) Internationale Anmeldenummer: PCT/EP2006/061134
(87) Internationale Veröffentlichungsnummer: WO 2006/103253

(56) Entgegenhaltungen:
- EP-A- 0 611 824
- GB-A- 2 235 457

### SEQUENCE LISTING

<110> BASF Aktiengesellschaft
<120> Verwendung von Proteinen als Demulgatoren
<130> AE 200500040
<160> 24
<170> PatentIn version 3.1
<210> 1
   <211> 405
   <212> DNA
   <213> basf-dewA
<220>
   <221> CDS
   <222> (1) .. (405)
   <223>
<400> 1
   atg cgc ttc atc gtc tct ctc ctc gcc ttc act gcc gcg gcc acc gcg        48
   Met Arg Phe Ile Val Ser Leu Leu Ala Phe Thr Ala Ala Ala Thr Ala
   1               5                   10                  15
   acc gcc ctc ccg gcc tct gcc gca aag aac gcg aag ctg gcc acc tcg        96
   Thr Ala Leu Pro Ala Ser Ala Ala Lys Asn Ala Lys Leu Ala Thr Ser
               20                  25                  30
   gcg gcc ttc gcc aag cag gct gaa ggc acc acc tgc aat gtc ggc tcg       144
   Ala Ala Phe Ala Lys Gln Ala Glu Gly Thr Thr Cys Asn Val Gly Ser
           35                  40                  45
   atc gct tgc tgc aac tcc ccc gct gag acc aac aac gac agt ctg ttg       192
   Ile Ala Cys Cys Asn Ser Pro Ala Glu Thr Asn Asn Asp Ser Leu Leu
       50                  55                  60
   agc ggt ctg ctc ggt gct ggc ctt ctc aac ggg ctc tcg ggc aac act       240
   Ser Gly Leu Leu Gly Ala Gly Leu Leu Asn Gly Leu Ser Gly Asn Thr
   65                  70                  75                  80
   ggc agc gcc tgc gcc aag gcg agc ttg att gac cag ctg ggt ctg ctc       288
   Gly Ser Ala Cys Ala Lys Ala Ser Leu Ile Asp Gln Leu Gly Leu Leu
                   85                  90                  95
   gct ctc gtc gac cac act gag gaa ggc ccc gtc tgc aag aac atc gtc       336
   Ala Leu Val Asp His Thr Glu Glu Gly Pro Val Cys Lys Asn Ile Val
               100                 105                 110
   gct tgc tgc cct gag gga acc acc aac tgt gtt gcc gtc gac aac gct       384
   Ala Cys Cys Pro Glu Gly Thr Thr Asn Cys Val Ala Val Asp Asn Ala
           115                 120                 125
   ggc gct ggt acc aag gct gag                                           405
   Gly Ala Gly Thr Lys Ala Glu
       130                 135
<210> 2
   <211> 135
   <212> PRT
   <213> basf-dewA
<400> 2
   Met Arg Phe Ile Val Ser Leu Leu Ala Phe Thr Ala Ala Ala Thr Ala
   1               5                   10                  15
   Thr Ala Leu Pro Ala Ser Ala Ala Lys Asn Ala Lys Leu Ala Thr Ser
               20                  25                  30
   Ala Ala Phe Ala Lys Gln Ala Glu Gly Thr Thr Cys Asn Val Gly Ser
           35                  40                  45
   Ile Ala Cys Cys Asn Ser Pro Ala Glu Thr Asn Asn Asp Ser Leu Leu
       50                  55                  60
   Ser Gly Leu Leu Gly Ala Gly Leu Leu Asn Gly Leu Ser Gly Asn Thr
   65                  70                  75                  80
   Gly Ser Ala Cys Ala Lys Ala Ser Leu Ile Asp Gln Leu Gly Leu Leu
                   85                  90                  95
   Ala Leu Val Asp His Thr Glu Glu Gly Pro Val Cys Lys Asn Ile Val
               100                 105                 110
   Ala Cys Cys Pro Glu Gly Thr Thr Asn Cys Val Ala Val Asp Asn Ala
           115                 120                 125
   Gly Ala Gly Thr Lys Ala Glu
       130                 135
<210> 3
   <211> 471
   <212> DNA
   <213> basf-rodA
<220>
   <221> CDS
   <222> (1) .. (471)
   <223>
<400> 3
   atg aag ttc tcc att gct gcc gct gtc gtt gct ttc gcc gcc tcc gtc        48
   Met Lys Phe Ser Ile Ala Ala Ala Val Val Ala Phe Ala Ala Ser Val
   1               5                   10                  15
   gcg gcc ctc cct cct gcc cat gat tcc cag ttc gct ggc aat ggt gtt        96
   Ala Ala Leu Pro Pro Ala His Asp Ser Gln Phe Ala Gly Asn Gly Val
               20                  25                  30
   ggc aac aag ggc aac agc aac gtc aag ttc cct gtc ccc gaa aac gtg       144
   Gly Asn Lys Gly Asn Ser Asn Val Lys Phe Pro Val Pro Glu Asn Val
           35                  40                  45
   acc gtc aag cag gcc tcc gac aag tgc ggt gac cag gcc cag ctc tct       192
   Thr Val Lys Gln Ala Ser Asp Lys Cys Gly Asp Gln Ala Gln Leu Ser
       50                  55                  60
   tgc tgc aac aag gcc acg tac gcc ggt gac acc aca acc gtt gat gag       240
   Cys Cys Asn Lys Ala Thr Tyr Ala Gly Asp Thr Thr Thr Val Asp Glu
   65                  70                  75                  80
   ggt ctt ctg tct ggt gcc ctc agc ggc ctc atc ggc gcc ggg tct ggt       288
   Gly Leu Leu Ser Gly Ala Leu Ser Gly Leu Ile Gly Ala Gly Ser Gly
                   85                  90                  95
   gcc gaa ggt ctt ggt ctc ttc gat cag tgc tcc aag ctt gat gtt gct       336
   Ala Glu Gly Leu Gly Leu Phe Asp Gln Cys Ser Lys Leu Asp Val Ala
               100                 105                 110
   gtc ctc att ggc atc caa gat ctt gtc aac cag aag tgc aag caa aac       384
   Val Leu Ile Gly Ile Gln Asp Leu Val Asn Gln Lys Cys Lys Gln Asn
           115                 120                 125
   att gcc tgc tgc cag aac tcc ccc tcc agc gcg gat ggc aac ctt att       432
   Ile Ala Cys Cys Gln Asn Ser Pro Ser Ser Ala Asp Gly Asn Leu Ile
       130                 135                 140
   ggt gtc ggt ctc cct tgc gtt gcc ctt ggc tcc atc ctc                   471
   Gly Val Gly Leu Pro Cys Val Ala Leu Gly Ser Ile Leu
   145                 150                 155
<210> 4
   <211> 157
   <212> PRT
   <213> basf-rodA
<400> 4
   Met Lys Phe Ser Ile Ala Ala Ala Val Val Ala Phe Ala Ala Ser Val
   1               5                   10                  15
   Ala Ala Leu Pro Pro Ala His Asp Ser Gln Phe Ala Gly Asn Gly Val
               20                  25                  30
   Gly Asn Lys Gly Asn Ser Asn Val Lys Phe Pro Val Pro Glu Asn Val
           35                  40                  45
   Thr Val Lys Gln Ala Ser Asp Lys Cys Gly Asp Gln Ala Gln Leu Ser
       50                  55                  60
   Cys Cys Asn Lys Ala Thr Tyr Ala Gly Asp Thr Thr Thr Val Asp Glu
   65                  70                  75                  80
   Gly Leu Leu Ser Gly Ala Leu Ser Gly Leu Ile Gly Ala Gly Ser Gly
                   85                  90                  95
   Ala Glu Gly Leu Gly Leu Phe Asp Gln Cys Ser Lys Leu Asp Val Ala
               100                 105                 110
   Val Leu Ile Gly Ile Gln Asp Leu Val Asn Gln Lys Cys Lys Gln Asn
           115                 120                 125
   Ile Ala Cys Cys Gln Asn Ser Pro Ser Ser Ala Asp Gly Asn Leu Ile
       130                 135                 140
   Gly Val Gly Leu Pro Cys Val Ala Leu Gly Ser Ile Leu
   145                 150                 155
<210> 5
   <211> 336
   <212> DNA
   <213> basf-HypA
   <220>
   <221> CDS
   <222> (1) .. (336)
   <223>
<400> 5
   atg atc tct cgc gtc ctt gtc gct gct ctc gtc gct ctc ccc gct ctt        48
   Met Ile Ser Arg Val Leu Val Ala Ala Leu Val Ala Leu Pro Ala Leu
   1               5                   10                  15
   gtt act gca act cct gct ccc gga aag cct aaa gcc agc agt cag tgc        96
   Val Thr Ala Thr Pro Ala Pro Gly Lys Pro Lys Ala Ser Ser Gln Cys
               20                  25                  30
   gac gtc ggt gaa atc cat tgc tgt gac act cag cag act ccc gac cac       144
   Asp Val Gly Glu Ile His Cys Cys Asp Thr Gln Gln Thr Pro Asp His
           35                  40                  45
   acc agc gcc gcc gcg tct ggt ttg ctt ggt gtt ccc atc aac ctt ggt       192
   Thr Ser Ala Ala Ala Ser Gly Leu Leu Gly Val Pro Ile Asn Leu Gly
       50                  55                  60
   gct ttc ctc ggt ttc gac tgt acc ccc att tcc gtc ctt ggc gtc ggt       240
   Ala Phe Leu Gly Phe Asp Cys Thr Pro Ile Ser Val Leu Gly Val Gly
   65                  70                  75                  80
   ggc aac aac tgt gct gct cag cct gtc tgc tgc aca gga aat caa ttc       288
   Gly Asn Asn Cys Ala Ala Gln Pro Val Cys Cys Thr Gly Asn Gln Phe
                   85                  90                  95
   acc gca ttg att aac gct ctt gac tgc tct cct gtc aat gtc aac ctc       336
   Thr Ala Leu Ile Asn Ala Leu Asp Cys Ser Pro Val Asn Val Asn Leu
               100                 105                 110
<210> 6
   <211> 112
   <212> PRT
   <213> basf-HypA
<400> 6
   Met Ile Ser Arg Val Leu Val Ala Ala Leu Val Ala Leu Pro Ala Leu
   1               5                   10                  15
   Val Thr Ala Thr Pro Ala Pro Gly Lys Pro Lys Ala Ser Ser Gln Cys
               20                  25                  30
   Asp Val Gly Glu Ile His Cys Cys Asp Thr Gln Gln Thr Pro Asp His
           35                  40                  45
   Thr Ser Ala Ala Ala Ser Gly Leu Leu Gly Val Pro Ile Asn Leu Gly
       50                  55                  60
   Ala Phe Leu Gly Phe Asp Cys Thr Pro Ile Ser Val Leu Gly Val Gly
   65                  70                  75                  80
   Gly Asn Asn Cys Ala Ala Gln Pro Val Cys Cys Thr Gly Asn Gln Phe
                   85                  90                  95
   Thr Ala Leu Ile Asn Ala Leu Asp Cys Ser Pro Val Asn Val Asn Leu
               100                 105                 110
<210> 7
   <211> 357
   <212> DNA
   <213> basf-HypB
<220>
   <221> CDS
   <222> (1) .. (357)
   <223>
<400> 7
   atg gtc agc acg ttc atc act gtc gca aag acc ctt ctc gtc gcg ctc      48
   Met Val Ser Thr Phe Ile Thr Val Ala Lys Thr Leu Leu Val Ala Leu
   1               5                   10                  15
   ctc ttc gtc aat atc aat atc gtc gtt ggt act gca act acc ggc aag      96
   Leu Phe Val Asn Ile Asn Ile Val Val Gly Thr Ala Thr Thr Gly Lys
               20                  25                  30
   cat tgt agc acc ggt cct atc gag tgc tgc aag cag gtc atg gat tct     144
   His Cys Ser Thr Gly Pro Ile Glu Cys Cys Lys Gln Val Met Asp Ser
           35                  40                  45
   aag agc cct cag gct acg gag ctt ctt acg aag aat ggc ctt ggc ctg     192
   Lys Ser Pro Gln Ala Thr Glu Leu Leu Thr Lys Asn Gly Leu Gly Leu
       50                  55                  60
   ggt gtc ctt gct ggc gtg aag ggt ctt gtt ggc gcg aat tgc agc cct     240
   Gly Val Leu Ala Gly Val Lys Gly Leu Val Gly Ala Asn Cys Ser Pro
   65                  70                  75                  80
   atc acg gca att ggt att ggc tcc ggc agc caa tgc tct ggc cag acc     288
   Ile Thr Ala Ile Gly Ile Gly Ser Gly Ser Gln Cys Ser Gly Gln Thr
                   85                  90                  95
   gtt tgc tgc cag aat aat aat ttc aac ggt gtt gtc gct att ggt tgc     336
   Val Cys Cys Gln Asn Asn Asn Phe Asn Gly Val Val Ala Ile Gly Cys
               100                 105                 110
   act ccc att aat gcc aat gtg                                         357
   Thr Pro Ile Asn Ala Asn Val
           115
<210> 8
   <211> 119
   <212> PRT
   <213> basf-HypB
<400> 8
   Met Val Ser Thr Phe Ile Thr Val Ala Lys Thr Leu Leu Val Ala Leu
   1               5                   10                  15
   Leu Phe Val Asn Ile Asn Ile Val Val Gly Thr Ala Thr Thr Gly Lys
               20                  25                  30
   His Cys Ser Thr Gly Pro Ile Glu Cys Cys Lys Gln Val Met Asp Ser
           35                  40                  45
   Lys Ser Pro Gln Ala Thr Glu Leu Leu Thr Lys Asn Gly Leu Gly Leu
       50                  55                  60
   Gly Val Leu Ala Gly Val Lys Gly Leu Val Gly Ala Asn Cys Ser Pro
   65                  70                  75                  80
   Ile Thr Ala Ile Gly Ile Gly Ser Gly Ser Gln Cys Ser Gly Gln Thr
                   85                  90                  95
   Val Cys Cys Gln Asn Asn Asn Phe Asn Gly Val Val Ala Ile Gly Cys
               100                 105                 110
   Thr Pro Ile Asn Ala Asn Val
           115
<210> 9
   <211> 408
   <212> DNA
   <213> basf-sc3
<220>
   <221> CDS
   <222> (1) .. (408)
   <223>
<400> 9
   atg ttc gcc cgt ctc ccc gtc gtg ttc ctc tac gcc ttc gtc gcg ttc       48
   Met Phe Ala Arg Leu Pro Val Val Phe Leu Tyr Ala Phe Val Ala Phe
   1               5                   10                  15
   ggc gcc ctc gtc gct gcc ctc cca ggt ggc cac ccg ggc acg acc acg       96
   Gly Ala Leu Val Ala Ala Leu Pro Gly Gly His Pro Gly Thr Thr Thr
               20                  25                  30
   ccg ccg gtt acg acg acg gtg acg gtg acc acg ccg ccc tcg acg acg      144
   Pro Pro Val Thr Thr Thr Val Thr Val Thr Thr Pro Pro Ser Thr Thr
           35                  40                  45
   acc atc gcc gcc ggt ggc acg tgt act acg ggg tcg ctc tct tgc tgc      192
   Thr Ile Ala Ala Gly Gly Thr Cys Thr Thr Gly Ser Leu Ser Cys Cys
       50                  55                  60
   aac cag gtt caa tcg gcg agc agc agc cct gtt acc gcc ctc ctc ggc      240
   Asn Gln Val Gln Ser Ala Ser Ser Ser Pro Val Thr Ala Leu Leu Gly
   65                  70                  75                  80
   ctg ctc ggc att gtc ctc agc gac ctc aac gtt ctc gtt ggc atc agc      288
   Leu Leu Gly Ile Val Leu Ser Asp Leu Asn Val Leu Val Gly Ile Ser
                   85                  90                  95
   tgc tct ccc ctc act gtc atc ggt gtc gga ggc agc ggc tgt tcg gcg      336
   Cys Ser Pro Leu Thr Val Ile Gly Val Gly Gly Ser Gly Cys Ser Ala
               100                 105                 110
   cag acc gtc tgc tgc gaa aac acc caa ttc aac ggg ctg atc aac atc      384
   Gln Thr Val Cys Cys Glu Asn Thr Gln Phe Asn Gly Leu Ile Asn Ile
           115                 120                 125
   ggt tgc acc ccc atc aac atc ctc                                      408
   Gly Cys Thr Pro Ile Asn Ile Leu
       130                 135
<210> 10
   <211> 136
   <212> PRT
   <213> basf-sc3
<400> 10
   Met Phe Ala Arg Leu Pro Val Val Phe Leu Tyr Ala Phe Val Ala Phe
   1               5                   10                  15
   Gly Ala Leu Val Ala Ala Leu Pro Gly Gly His Pro Gly Thr Thr Thr
               20                  25                  30
   Pro Pro Val Thr Thr Thr Val Thr Val Thr Thr Pro Pro Ser Thr Thr
           35                  40                  45
   Thr Ile Ala Ala Gly Gly Thr Cys Thr Thr Gly Ser Leu Ser Cys Cys
       50                  55                  60
   Asn Gln Val Gln Ser Ala Ser Ser Ser Pro Val Thr Ala Leu Leu Gly
   65                  70                  75                  80
   Leu Leu Gly Ile Val Leu Ser Asp Leu Asn Val Leu Val Gly Ile Ser
                   85                  90                  95
   Cys Ser Pro Leu Thr Val Ile Gly Val Gly Gly Ser Gly Cys Ser Ala
               100                 105                 110
   Gln Thr Val Cys Cys Glu Asn Thr Gln Phe Asn Gly Leu Ile Asn Ile
           115                 120                 125
   Gly Cys Thr Pro Ile Asn Ile Leu
       130                 135
<210> 11
   <211> 483
   <212> DNA
   <213> basf-BASF1
<220>
   <221> CDS
   <222> (1)..(483)
   <223>
<400> 11
   atg aag ttc tcc gtc tcc gcc gcc gtc ctc gcc ttc gcc gcc tcc gtc       48
   Met Lys Phe Ser Val Ser Ala Ala Val Leu Ala Phe Ala Ala Ser Val
   1               5                   10                  15
   gcc gcc ctc cct cag cac gac tcc gcc gcc ggc aac ggc aac ggc gtc       96
   Ala Ala Leu Pro Gln His Asp Ser Ala Ala Gly Asn Gly Asn Gly Val
               20                  25                  30
   ggc aac aag ttc cct gtc cct gac gac gtc acc gtc aag cag gcc acc      144
   Gly Asn Lys Phe Pro Val Pro Asp Asp Val Thr Val Lys Gln Ala Thr
           35                  40                  45
   gac aag tgc ggc gac cag gcc cag ctc tcc tgc tgc aac aag gcc acc      192
   Asp Lys Cys Gly Asp Gln Ala Gln Leu Ser Cys Cys Asn Lys Ala Thr
       50                  55                  60
   tac gcc ggc gac gtc ctc acc gac atc gac gag ggc atc ctc gcc ggc      240
   Tyr Ala Gly Asp Val Leu Thr Asp Ile Asp Glu Gly Ile Leu Ala Gly
   65                  70                  75                  80
   ctc ctc aag aac ctc atc ggc ggc ggc tcc ggc tcc gag ggc ctc ggc      288
   Leu Leu Lys Asn Leu Ile Gly Gly Gly Ser Gly Ser Glu Gly Leu Gly
                   85                  90                  95
   ctc ttc gac cag tgc gtc aag ctc gac ctc cag atc tcc gtc atc ggc      336
   Leu Phe Asp Gln Cys Val Lys Leu Asp Leu Gln Ile Ser Val Ile Gly
               100                 105                 110
   atc cct atc cag gac ctc ctc aac cag gtc aac aag cag tgc aag cag      384
   Ile Pro Ile Gln Asp Leu Leu Asn Gln Val Asn Lys Gln Cys Lys Gln
           115                 120                 125
   aac atc gcc tgc tgc cag aac tcc cct tcc gac gcc acc ggc tcc ctc      432
   Asn Ile Ala Cys Cys Gln Asn Ser Pro Ser Asp Ala Thr Gly Ser Leu
       130                 135                 140
   gtc aac ctc ggc ctc ggc aac cct tgc atc cct gtc tcc ctc ctc cat      480
   Val Asn Leu Gly Leu Gly Asn Pro Cys Ile Pro Val Ser Leu Leu His
   145                 150                 155                 160
   atg                                                                  483
   Met
<210> 12
   <211> 161
   <212> PRT
   <213> basf-BASF1
<400> 12
   Met Lys Phe Ser Val Ser Ala Ala Val Leu Ala Phe Ala Ala Ser Val
   1               5                   10                  15
   Ala Ala Leu Pro Gln His Asp Ser Ala Ala Gly Asn Gly Asn Gly Val
               20                  25                  30
   Gly Asn Lys Phe Pro Val Pro Asp Asp Val Thr Val Lys Gln Ala Thr
           35                  40                  45
   Asp Lys Cys Gly Asp Gln Ala Gln Leu Ser Cys Cys Asn Lys Ala Thr
       50                  55                  60
   Tyr Ala Gly Asp Val Leu Thr Asp Ile Asp Glu Gly Ile Leu Ala Gly
   65                  70                  75                  80
   Leu Leu Lys Asn Leu Ile Gly Gly Gly Ser Gly Ser Glu Gly Leu Gly
                   85                  90                  95
   Leu Phe Asp Gln Cys Val Lys Leu Asp Leu Gln Ile Ser Val Ile Gly
               100                 105                 110
   Ile Pro Ile Gln Asp Leu Leu Asn Gln Val Asn Lys Gln Cys Lys Gln
           115                 120                 125
   Asn Ile Ala Cys Cys Gln Asn Ser Pro Ser Asp Ala Thr Gly Ser Leu
       130                 135                 140
   Val Asn Leu Gly Leu Gly Asn Pro Cys Ile Pro Val Ser Leu Leu His
   145                 150                 155                 160
   Met
<210> 13
   <211> 465
   <212> DNA
   <213> basf-BASF2
<220>
   <221> CDS
   <222> (1)..(465)
   <223>
<400> 13
   atg aag ttc tcc gtc tcc gcc gcc gtc ctc gcc ttc gcc gcc tcc gtc      48
   Met Lys Phe Ser Val Ser Ala Ala Val Leu Ala Phe Ala Ala Ser Val
   1               5                   10                  15
   gcc gcc ctc cct cag cac gac tcc gcc gcc ggc aac ggc aac ggc gtc      96
   Ala Ala Leu Pro Gln His Asp Ser Ala Ala Gly Asn Gly Asn Gly Val
               20                  25                  30
   ggc aac aag ttc cct gtc cct gac gac gtc acc gtc aag cag gcc acc      144
   Gly Asn Lys Phe Pro Val Pro Asp Asp Val Thr Val Lys Gln Ala Thr
           35                  40                  45
   gac aag tgc ggc gac cag gcc cag ctc tcc tgc tgc aac aag gcc acc      192
   Asp Lys Cys Gly Asp Gln Ala Gln Leu Ser Cys Cys Asn Lys Ala Thr
       50                  55                  60
   tac gcc ggc gac gtc acc gac atc gac gag ggc atc ctc gcc ggc ctc      240
   Tyr Ala Gly Asp Val Thr Asp Ile Asp Glu Gly Ile Leu Ala Gly Leu
   65                  70                  75                  80
   ctc aag aac ctc atc ggc ggc ggc tcc ggc tcc gag ggc ctc ggc ctc      288
   Leu Lys Asn Leu Ile Gly Gly Gly Ser Gly Ser Glu Gly Leu Gly Leu
                   85                  90                  95
   ttc gac cag tgc gtc aag ctc gac ctc cag atc tcc gtc atc ggc atc      336
   Phe Asp Gln Cys Val Lys Leu Asp Leu Gln Ile Ser Val Ile Gly Ile
               100                 105                 110
   cct atc cag gac ctc ctc aac cag cag tgc aag cag aac atc gcc tgc      384
   Pro Ile Gln Asp Leu Leu Asn Gln Gln Cys Lys Gln Asn Ile Ala Cys
           115                 120                 125
   tgc cag aac tcc cct tcc gac gcc acc ggc tcc ctc gtc aac ctc ggc      432
   Cys Gln Asn Ser Pro Ser Asp Ala Thr Gly Ser Leu Val Asn Leu Gly
       130                 135                 140
   aac cct tgc atc cct gtc tcc ctc ctc cat atg                          465
   Asn Pro Cys Ile Pro Val Ser Leu Leu His Met
   145                 150                 155
<210> 14
   <211> 155
   <212> PRT
   <213> basf-BASF2
<400> 14
   Met Lys Phe Ser Val Ser Ala Ala Val Leu Ala Phe Ala Ala Ser Val
   1               5                   10                  15
   Ala Ala Leu Pro Gln His Asp Ser Ala Ala Gly Asn Gly Asn Gly Val
               20                  25                  30
   Gly Asn Lys Phe Pro Val Pro Asp Asp Val Thr Val Lys Gln Ala Thr
           35                  40                  45
   Asp Lys Cys Gly Asp Gln Ala Gln Leu Ser Cys Cys Asn Lys Ala Thr
       50                  55                  60
   Tyr Ala Gly Asp Val Thr Asp Ile Asp Glu Gly Ile Leu Ala Gly Leu
   65                  70                  75                  80
   Leu Lys Asn Leu Ile Gly Gly Gly Ser Gly Ser Glu Gly Leu Gly Leu
                   85                  90                  95
   Phe Asp Gln Cys Val Lys Leu Asp Leu Gln Ile Ser Val Ile Gly Ile
               100                 105                 110
   Pro Ile Gln Asp Leu Leu Asn Gln Gln Cys Lys Gln Asn Ile Ala Cys
           115                 120                 125
   Cys Gln Asn Ser Pro Ser Asp Ala Thr Gly Ser Leu Val Asn Leu Gly
       130                 135                 140
   Asn Pro Cys Ile Pro Val Ser Leu Leu His Met
   145                 150                 155
<210> 15
   <211> 882
   <212> DNA
   <213> basf-yaad
<220>
   <221> CDS
   <222> (1)..(882)
   <223>
<400> 15
   atg gct caa aca ggt act gaa cgt gta aaa cgc gga atg gca gaa atg      48
   Met Ala Gln Thr Gly Thr Glu Arg Val Lys Arg Gly Met Ala Glu Met
   1               5                   10                  15
   caa aaa ggc ggc gtc atc atg gac gtc atc aat gcg gaa caa gcg aaa      96
   Gln Lys Gly Gly Val Ile Met Asp Val Ile Asn Ala Glu Gln Ala Lys
               20                  25                  30
   atc gct gaa gaa gct gga gct gtc gct gta atg gcg cta gaa cgt gtg     144
   Ile Ala Glu Glu Ala Gly Ala Val Ala Val Met Ala Leu Glu Arg Val
           35                  40                  45
   cca gca gat att cgc gcg gct gga gga gtt gcc cgt atg gct gac cct     192
   Pro Ala Asp Ile Arg Ala Ala Gly Gly Val Ala Arg Met Ala Asp Pro
       50                  55                  60
   aca atc gtg gaa gaa gta atg aat gca gta tct atc ccg gta atg gca     240
   Thr Ile Val Glu Glu Val Met Asn Ala Val Ser Ile Pro Val Met Ala
   65                  70                  75                  80
   aaa gcg cgt atc gga cat att gtt gaa gcg cgt gtg ctt gaa gct atg     288
   Lys Ala Arg Ile Gly His Ile Val Glu Ala Arg Val Leu Glu Ala Met
                   85                  90                  95
   ggt gtt gac tat att gat gaa agt gaa gtt ctg acg ccg gct gac gaa     336
   Gly Val Asp Tyr Ile Asp Glu Ser Glu Val Leu Thr Pro Ala Asp Glu
               100                 105                 110
   gaa ttt cat tta aat aaa aat gaa tac aca gtt cct ttt gtc tgt ggc     384
   Glu Phe His Leu Asn Lys Asn Glu Tyr Thr Val Pro Phe Val Cys Gly
           115                 120                 125
   tgc cgt gat ctt ggt gaa gca aca cgc cgt att gcg gaa ggt gct tct     432
   Cys Arg Asp Leu Gly Glu Ala Thr Arg Arg Ile Ala Glu Gly Ala Ser
       130                 135                 140
   atg ctt cgc aca aaa ggt gag cct gga aca ggt aat att gtt gag gct     480
   Met Leu Arg Thr Lys Gly Glu Pro Gly Thr Gly Asn Ile Val Glu Ala
   145                 150                 155                 160
   gtt cgc cat atg cgt aaa gtt aac gct caa gtg cgc aaa gta gtt gcg     528
   Val Arg His Met Arg Lys Val Asn Ala Gln Val Arg Lys Val Val Ala
                   165                 170                 175
   atg agt gag gat gag cta atg aca gaa gcg aaa aac cta ggt gct cct     576
   Met Ser Glu Asp Glu Leu Met Thr Glu Ala Lys Asn Leu Gly Ala Pro
               180                 185                 190
   tac gag ctt ctt ctt caa att aaa aaa gac ggc aag ctt cct gtc gtt     624
   Tyr Glu Leu Leu Leu Gln Ile Lys Lys Asp Gly Lys Leu Pro Val Val
           195                 200                 205
   aac ttt gcc gct ggc ggc gta gca act cca gct gat gct gct ctc atg     672
   Asn Phe Ala Ala Gly Gly Val Ala Thr Pro Ala Asp Ala Ala Leu Met
       210                 215                 220
   atg cag ctt ggt gct gac gga gta ttt gtt ggt tct ggt att ttt aaa     720
   Met Gln Leu Gly Ala Asp Gly Val Phe Val Gly Ser Gly Ile Phe Lys
   225                 230                 235                 240
   tca gac aac cct gct aaa ttt gcg aaa gca att gtg gaa gca aca act     768
   Ser Asp Asn Pro Ala Lys Phe Ala Lys Ala Ile Val Glu Ala Thr Thr
                   245                 250                 255
   cac ttt act gat tac aaa tta atc gct gag ttg tca aaa gag ctt ggt      816
   His Phe Thr Asp Tyr Lys Leu Ile Ala Glu Leu Ser Lys Glu Leu Gly
               260                     265                     270
   act gca atg aaa ggg att gaa atc tca aac tta ctt cca gaa cag cgt      864
   Thr Ala Met Lys Gly Ile Glu Ile Ser Asn Leu Leu Pro Glu Gln Arg
           275                     280                     285
   atg caa gaa cgc ggc tgg                                              882
   Met Gln Glu Arg Gly Trp
       290
<210> 16
   <211> 294
   <212> PRT
   <213> basf-yaad
<400> 16
   Met Ala Gln Thr Gly Thr Glu Arg Val Lys Arg Gly Met Ala Glu Met
   1               5                   10                  15
   Gln Lys Gly Gly Val Ile Met Asp Val Ile Asn Ala Glu Gln Ala Lys
               20                  25                  30
   Ile Ala Glu Glu Ala Gly Ala Val Ala Val Met Ala Leu Glu Arg Val
           35                  40                  45
   Pro Ala Asp Ile Arg Ala Ala Gly Gly Val Ala Arg Met Ala Asp Pro
       50                  55                  60
   Thr Ile Val Glu Glu Val Met Asn Ala Val Ser Ile Pro Val Met Ala
   65                  70                  75                  80
   Lys Ala Arg Ile Gly His Ile Val Glu Ala Arg Val Leu Glu Ala Met
                   85                  90                  95
   Gly Val Asp Tyr Ile Asp Glu Ser Glu Val Leu Thr Pro Ala Asp Glu
               100                 105                 110
   Glu Phe His Leu Asn Lys Asn Glu Tyr Thr Val Pro Phe Val Cys Gly
           115                 120                 125
   Cys Arg Asp Leu Gly Glu Ala Thr Arg Arg Ile Ala Glu Gly Ala Ser
       130                 135                 140
   Met Leu Arg Thr Lys Gly Glu Pro Gly Thr Gly Asn Ile Val Glu Ala
   145                 150                 155                 160
   Val Arg His Met Arg Lys Val Asn Ala Gln Val Arg Lys Val Val Ala
                   165                 170                 175
   Met Ser Glu Asp Glu Leu Met Thr Glu Ala Lys Asn Leu Gly Ala Pro
               180                 185                 190
   Tyr Glu Leu Leu Leu Gln Ile Lys Lys Asp Gly Lys Leu Pro Val Val
           195                 200                 205
   Asn Phe Ala Ala Gly Gly Val Ala Thr Pro Ala Asp Ala Ala Leu Met
       210                 215                 220
   Met Gln Leu Gly Ala Asp Gly Val Phe Val Gly Ser Gly Ile Phe Lys
   225                 230                 235                 240
   Ser Asp Asn Pro Ala Lys Phe Ala Lys Ala Ile Val Glu Ala Thr Thr
                   245                 250                 255
   His Phe Thr Asp Tyr Lys Leu Ile Ala Glu Leu Ser Lys Glu Leu Gly
               260                 265                 270
   Thr Ala Met Lys Gly Ile Glu Ile Ser Asn Leu Leu Pro Glu Gln Arg
           275                 280                 285
   Met Gln Glu Arg Gly Trp
       290
<210> 17
   <211> 591
   <212> DNA
   <213> basf-yaae
<220>
   <221> CDS
   <222> (1)..(591)
   <223>
<400> 17
   atg gga tta aca ata ggt gta cta gga ctt caa gga gca gtt aga gag      48
   Met Gly Leu Thr Ile Gly Val Leu Gly Leu Gln Gly Ala Val Arg Glu
   1               5                   10                  15
   cac atc cat gcg att gaa gca tgc ggc gcg gct ggt ctt gtc gta aaa      96
   His Ile His Ala Ile Glu Ala Cys Gly Ala Ala Gly Leu Val Val Lys
               20                  25                  30
   cgt ccg gag cag ctg aac gaa gtt gac ggg ttg att ttg ccg ggc ggt      144
   Arg Pro Glu Gln Leu Asn Glu Val Asp Gly Leu Ile Leu Pro Gly Gly
           35                  40                  45
   gag agc acg acg atg cgc cgt ttg atc gat acg tat caa ttc atg gag      192
   Glu Ser Thr Thr Met Arg Arg Leu Ile Asp Thr Tyr Gln Phe Met Glu
       50                  55                  60
   ccg ctt cgt gaa ttc gct gct cag ggc aaa ccg atg ttt gga aca tgt      240
   Pro Leu Arg Glu Phe Ala Ala Gln Gly Lys Pro Met Phe Gly Thr Cys
   65                  70                  75                  80
   gcc gga tta att ata tta gca aaa gaa att gcc ggt tca gat aat cct      288
   Ala Gly Leu Ile Ile Leu Ala Lys Glu Ile Ala Gly Ser Asp Asn Pro
                   85                  90                  95
   cat tta ggt ctt ctg aat gtg gtt gta gaa cgt aat tca ttt ggc cgg      336
   His Leu Gly Leu Leu Asn Val Val Val Glu Arg Asn Ser Phe Gly Arg
               100                 105                 110
   cag gtt gac agc ttt gaa gct gat tta aca att aaa ggc ttg gac gag      384
   Gln Val Asp Ser Phe Glu Ala Asp Leu Thr Ile Lys Gly Leu Asp Glu
           115                 120                 125
   cct ttt act ggg gta ttc atc cgt gct ccg cat att tta gaa gct ggt      432
   Pro Phe Thr Gly Val Phe Ile Arg Ala Pro His Ile Leu Glu Ala Gly
       130                 135                 140
   gaa aat gtt gaa gtt cta tcg gag cat aat ggt cgt att gta gcc gcg      480
   Glu Asn Val Glu Val Leu Ser Glu His Asn Gly Arg Ile Val Ala Ala
   145                 150                 155                 160
   aaa cag ggg caa ttc ctt ggc tgc tca ttc cat ccg gag ctg aca gaa      528
   Lys Gln Gly Gln Phe Leu Gly Cys Ser Phe His Pro Glu Leu Thr Glu
                   165                 170                 175
   gat cac cga gtg acg cag ctg ttt gtt gaa atg gtt gag gaa tat aag      576
   Asp His Arg Val Thr Gln Leu Phe Val Glu Met Val Glu Glu Tyr Lys
               180                 185                 190
   caa aag gca ctt gta                                                  591
   Gln Lys Ala Leu Val
           195
<210> 18
   <211> 197
   <212> PRT
   <213> basf-yaae
<400> 18
   Met Gly Leu Thr Ile Gly Val Leu Gly Leu Gln Gly Ala Val Arg Glu
   1               5                   10                  15
   His Ile His Ala Ile Glu Ala Cys Gly Ala Ala Gly Leu Val Val Lys
               20                  25                  30
   Arg Pro Glu Gln Leu Asn Glu Val Asp Gly Leu Ile Leu Pro Gly Gly
           35                  40                  45
   Glu Ser Thr Thr Met Arg Arg Leu Ile Asp Thr Tyr Gln Phe Met Glu
       50                  55                  60
   Pro Leu Arg Glu Phe Ala Ala Gln Gly Lys Pro Met Phe Gly Thr Cys
   65                  70                  75                  80
   Ala Gly Leu Ile Ile Leu Ala Lys Glu Ile Ala Gly Ser Asp Asn Pro
                   85                  90                  95
   His Leu Gly Leu Leu Asn Val Val Val Glu Arg Asn Ser Phe Gly Arg
               100                 105                 110
   Gln Val Asp Ser Phe Glu Ala Asp Leu Thr Ile Lys Gly Leu Asp Glu
           115                 120                 125
   Pro Phe Thr Gly Val Phe Ile Arg Ala Pro His Ile Leu Glu Ala Gly
       130                 135                 140
   Glu Asn Val Glu Val Leu Ser Glu His Asn Gly Arg Ile Val Ala Ala
   145                 150                 155                 160
   Lys Gln Gly Gln Phe Leu Gly Cys Ser Phe His Pro Glu Leu Thr Glu
                   165                 170                 175
   Asp His Arg Val Thr Gln Leu Phe Val Glu Met Val Glu Glu Tyr Lys
               180                 185                 190
   Gln Lys Ala Leu Val
           195
<210> 19
   <211> 1329
   <212> DNA
   <213> basf-yaad-Xa-dewA-his
<220>
   <221> CDS
   <222> (1)..(1329)
   <223>
<400> 19
   atg gct caa aca ggt act gaa cgt gta aaa cgc gga atg gca gaa atg       48
   Met Ala Gln Thr Gly Thr Glu Arg Val Lys Arg Gly Met Ala Glu Met
   1               5                   10                  15
   caa aaa ggc ggc gtc atc atg gac gtc atc aat gcg gaa caa gcg aaa       96
   Gln Lys Gly Gly Val Ile Met Asp Val Ile Asn Ala Glu Gln Ala Lys
               20                  25                  30
   atc gct gaa gaa gct gga gct gtc gct gta atg gcg cta gaa cgt gtg      144
   Ile Ala Glu Glu Ala Gly Ala Val Ala Val Met Ala Leu Glu Arg Val
           35                  40                  45
   cca gca gat att cgc gcg gct gga gga gtt gcc cgt atg gct gac cct      192
   Pro Ala Asp Ile Arg Ala Ala Gly Gly Val Ala Arg Met Ala Asp Pro
       50                  55                  60
   aca atc gtg gaa gaa gta atg aat gca gta tct atc ccg gta atg gca      240
   Thr Ile Val Glu Glu Val Met Asn Ala Val Ser Ile Pro Val Met Ala
   65                  70                  75                  80
   aaa gcg cgt atc gga cat att gtt gaa gcg cgt gtg ctt gaa gct atg      288
   Lys Ala Arg Ile Gly His Ile Val Glu Ala Arg Val Leu Glu Ala Met
                   85                  90                  95
   ggt gtt gac tat att gat gaa agt gaa gtt ctg acg ccg gct gac gaa      336
   Gly Val Asp Tyr Ile Asp Glu Ser Glu Val Leu Thr Pro Ala Asp Glu
               100                 105                 110
   gaa ttt cat tta aat aaa aat gaa tac aca gtt cct ttt gtc tgt ggc      384
   Glu Phe His Leu Asn Lys Asn Glu Tyr Thr Val Pro Phe Val Cys Gly
           115                 120                 125
   tgc cgt gat ctt ggt gaa gca aca cgc cgt att gcg gaa ggt gct tct      432
   Cys Arg Asp Leu Gly Glu Ala Thr Arg Arg Ile Ala Glu Gly Ala Ser
       130                 135                 140
   atg ctt cgc aca aaa ggt gag cct gga aca ggt aat att gtt gag gct      480
   Met Leu Arg Thr Lys Gly Glu Pro Gly Thr Gly Asn Ile Val Glu Ala
   145                 150                 155                 160
   gtt cgc cat atg cgt aaa gtt aac gct caa gtg cgc aaa gta gtt gcg      528
   Val Arg His Met Arg Lys Val Asn Ala Gln Val Arg Lys Val Val Ala
                   165                 170                 175
   atg agt gag gat gag cta atg aca gaa gcg aaa aac cta ggt gct cct      576
   Met Ser Glu Asp Glu Leu Met Thr Glu Ala Lys Asn Leu Gly Ala Pro
               180                 185                 190
   tac gag ctt ctt ctt caa att aaa aaa gac ggc aag ctt cct gtc gtt      624
   Tyr Glu Leu Leu Leu Gln Ile Lys Lys Asp Gly Lys Leu Pro Val Val
           195                 200                 205
   aac ttt gcc gct ggc ggc gta gca act cca gct gat gct gct ctc atg      672
   Asn Phe Ala Ala Gly Gly Val Ala Thr Pro Ala Asp Ala Ala Leu Met
       210                 215                 220
   atg cag ctt ggt gct gac gga gta ttt gtt ggt tct ggt att ttt aaa      720
   Met Gln Leu Gly Ala Asp Gly Val Phe Val Gly Ser Gly Ile Phe Lys
   225                 230                 235                 240
   tca gac aac cct gct aaa ttt gcg aaa gca att gtg gaa gca aca act      768
   Ser Asp Asn Pro Ala Lys Phe Ala Lys Ala Ile Val Glu Ala Thr Thr
                   245                 250                 255
   cac ttt act gat tac aaa tta atc gct gag ttg tca aaa gag ctt ggt      816
   His Phe Thr Asp Tyr Lys Leu Ile Ala Glu Leu Ser Lys Glu Leu Gly
               260                 265                 270
   act gca atg aaa ggg att gaa atc tca aac tta ctt cca gaa cag cgt      864
   Thr Ala Met Lys Gly Ile Glu Ile Ser Asn Leu Leu Pro Glu Gln Arg
           275                 280                 285
   atg caa gaa cgc ggc tgg aga tcc att gaa ggc cgc atg cgc ttc atc      912
   Met Gln Glu Arg Gly Trp Arg Ser Ile Glu Gly Arg Met Arg Phe Ile
       290                 295                 300
   gtc tct ctc ctc gcc ttc act gcc gcg gcc acc gcg acc gcc ctc ccg      960
   Val Ser Leu Leu Ala Phe Thr Ala Ala Ala Thr Ala Thr Ala Leu Pro
   305                 310                 315                 320
   gcc tct gcc gca aag aac gcg aag ctg gcc acc tcg gcg gcc ttc gcc     1008
   Ala Ser Ala Ala Lys Asn Ala Lys Leu Ala Thr Ser Ala Ala Phe Ala
                   325                 330                 335
   aag cag gct gaa ggc acc acc tgc aat gtc ggc tcg atc gct tgc tgc     1056
   Lys Gln Ala Glu Gly Thr Thr Cys Asn Val Gly Ser Ile Ala Cys Cys
               340                 345                 350
   aac tcc ccc gct gag acc aac aac gac agt ctg ttg agc ggt ctg ctc     1104
   Asn Ser Pro Ala Glu Thr Asn Asn Asp Ser Leu Leu Ser Gly Leu Leu
           355                 360                 365
   ggt gct ggc ctt ctc aac ggg ctc tcg ggc aac act ggc agc gcc tgc     1152
   Gly Ala Gly Leu Leu Asn Gly Leu Ser Gly Asn Thr Gly Ser Ala Cys
       370                 375                 380
   gcc aag gcg agc ttg att gac cag ctg ggt ctg ctc gct ctc gtc gac     1200
   Ala Lys Ala Ser Leu Ile Asp Gln Leu Gly Leu Leu Ala Leu Val Asp
   385                 390                 395                 400
   cac act gag gaa ggc ccc gtc tgc aag aac atc gtc gct tgc tgc cct     1248
   His Thr Glu Glu Gly Pro Val Cys Lys Asn Ile Val Ala Cys Cys Pro
                   405                 410                 415
   gag gga acc acc aac tgt gtt gcc gtc gac aac gct ggc gct ggt acc     1296
   Glu Gly Thr Thr Asn Cys Val Ala Val Asp Asn Ala Gly Ala Gly Thr
               420                 425                 430
   aag gct gag gga tct cat cac cat cac cat cac                         1329
   Lys Ala Glu Gly Ser His His His His His His
           435                 440
<210> 20
   <211> 443
   <212> PRT
   <213> basf-yaad-Xa-dewA-his
<400> 20
   Met Ala Gln Thr Gly Thr Glu Arg Val Lys Arg Gly Met Ala Glu Met
   1               5                   10                  15
   Gln Lys Gly Gly Val Ile Met Asp Val Ile Asn Ala Glu Gln Ala Lys
   20 25 30
   Ile Ala Glu Glu Ala Gly Ala Val Ala Val Met Ala Leu Glu Arg Val
   35 40 45
   Pro Ala Asp Ile Arg Ala Ala Gly Gly Val Ala Arg Met Ala Asp Pro
   50 55 60
   Thr Ile Val Glu Glu Val Met Asn Ala Val Ser Ile Pro Val Met Ala
   65 70 75 80
   Lys Ala Arg Ile Gly His Ile Val Glu Ala Arg Val Leu Glu Ala Met
   85 90 95
   Gly Val Asp Tyr Ile Asp Glu Ser Glu Val Leu Thr Pro Ala Asp Glu
   100 105 110
   Glu Phe His Leu Asn Lys Asn Glu Tyr Thr Val Pro Phe Val Cys Gly
   115 120 125
   Cys Arg Asp Leu Gly Glu Ala Thr Arg Arg Ile Ala Glu Gly Ala Ser
   130 135 140
   Met Leu Arg Thr Lys Gly Glu Pro Gly Thr Gly Asn Ile Val Glu Ala
   145 150 155 160
   Val Arg His Met Arg Lys Val Asn Ala Gln Val Arg Lys Val Val Ala
   165 170 175
   Met Ser Glu Asp Glu Leu Met Thr Glu Ala Lys Asn Leu Gly Ala Pro
   180 185 190
   Tyr Glu Leu Leu Leu Gln Ile Lys Lys Asp Gly Lys Leu Pro Val Val
   195 200 205
   Asn Phe Ala Ala Gly Gly Val Ala Thr Pro Ala Asp Ala Ala Leu Met
   210 215 220
   Met Gln Leu Gly Ala Asp Gly Val Phe Val Gly Ser Gly Ile Phe Lys
   225 230 235 240
   Ser Asp Asn Pro Ala Lys Phe Ala Lys Ala Ile Val Glu Ala Thr Thr
   245 250 255
   His Phe Thr Asp Tyr Lys Leu Ile Ala Glu Leu Ser Lys Glu Leu Gly
   260 265 270
   Thr Ala Met Lys Gly Ile Glu Ile Ser Asn Leu Leu Pro Glu Gln Arg
   275 280 285
   Met Gln Glu Arg Gly Trp Arg Ser Ile Glu Gly Arg Met Arg Phe Ile
   290 295 300
   Val Ser Leu Leu Ala Phe Thr Ala Ala Ala Thr Ala Thr Ala Leu Pro
   305 310 315 320
   Ala Ser Ala Ala Lys Asn Ala Lys Leu Ala Thr Ser Ala Ala Phe Ala
   325 330 335
   Lys Gln Ala Glu Gly Thr Thr Cys Asn Val Gly Ser Ile Ala Cys Cys
   340 345 350
   Asn Ser Pro Ala Glu Thr Asn Asn Asp Ser Leu Leu Ser Gly Leu Leu
   355 360 365
   Gly Ala Gly Leu Leu Asn Gly Leu Ser Gly Asn Thr Gly Ser Ala Cys
   370 375 380
   Ala Lys Ala Ser Leu Ile Asp Gln Leu Gly Leu Leu Ala Leu Val Asp
   385 390 395 400
   His Thr Glu Glu Gly Pro Val Cys Lys Asn Ile Val Ala Cys Cys Pro
   405 410 415
   Glu Gly Thr Thr Asn Cys Val Ala Val Asp Asn Ala Gly Ala Gly Thr
   420 425 430
   Lys Ala Glu Gly Ser His His His His His His
   435 440
<210> 21
   <211> 1395
   <212> DNA
   <213> basf-yaad-Xa-rodA-his
<220>
   <221> CDS
   <222> (1)..(1395)
<223>
<400> 21
   atg gct caa aca ggt act gaa cgt gta aaa cgc gga atg gca gaa atg 48
   Met Ala Gln Thr Gly Thr Glu Arg Val Lys Arg Gly Met Ala Glu Met
   1               5                   10                  15
   caa aaa ggc ggc gtc atc atg gac gtc atc aat gcg gaa caa gcg aaa      96
   Gln Lys Gly Gly Val Ile Met Asp Val Ile Asn Ala Glu Gln Ala Lys
               20                  25                  30
   atc gct gaa gaa gct gga gct gtc gct gta atg gcg cta gaa cgt gtg     144
   Ile Ala Glu Glu Ala Gly Ala Val Ala Val Met Ala Leu Glu Arg Val
           35                  40                  45
   cca gca gat att cgc gcg gct gga gga gtt gcc cgt atg gct gac cct     192
   Pro Ala Asp Ile Arg Ala Ala Gly Gly Val Ala Arg Met Ala Asp Pro
       50                  55                  60
   aca atc gtg gaa gaa gta atg aat gca gta tct atc ccg gta atg gca     240
   Thr Ile Val Glu Glu Val Met Asn Ala Val Ser Ile Pro Val Met Ala
   65                  70                  75                  80
   aaa gcg cgt atc gga cat att gtt gaa gcg cgt gtg ctt gaa gct atg     288
   Lys Ala Arg Ile Gly His Ile Val Glu Ala Arg Val Leu Glu Ala Met
                   85                  90                  95
   ggt gtt gac tat att gat gaa agt gaa gtt ctg acg ccg gct gac gaa     336
   Gly Val Asp Tyr Ile Asp Glu Ser Glu Val Leu Thr Pro Ala Asp Glu
               100                 105                 110
   gaa ttt cat tta aat aaa aat gaa tac aca gtt cct ttt gtc tgt ggc     384
   Glu Phe His Leu Asn Lys Asn Glu Tyr Thr Val Pro Phe Val Cys Gly
           115                 120                 125
   tgc cgt gat ctt ggt gaa gca aca cgc cgt att gcg gaa ggt gct tct     432
   Cys Arg Asp Leu Gly Glu Ala Thr Arg Arg Ile Ala Glu Gly Ala Ser
       130                 135                 140
   atg ctt cgc aca aaa ggt gag cct gga aca ggt aat att gtt gag gct     480
   Met Leu Arg Thr Lys Gly Glu Pro Gly Thr Gly Asn Ile Val Glu Ala
   145                 150                 155                 160
   gtt cgc cat atg cgt aaa gtt aac gct caa gtg cgc aaa gta gtt gcg     528
   Val Arg His Met Arg Lys Val Asn Ala Gln Val Arg Lys Val Val Ala
                   165                 170                 175
   atg agt gag gat gag cta atg aca gaa gcg aaa aac cta ggt gct cct     576
   Met Ser Glu Asp Glu Leu Met Thr Glu Ala Lys Asn Leu Gly Ala Pro
               180                 185                 190
   tac gag ctt ctt ctt caa att aaa aaa gac ggc aag ctt cct gtc gtt     624
   Tyr Glu Leu Leu Leu Gln Ile Lys Lys Asp Gly Lys Leu Pro Val Val
           195                 200                 205
   aac ttt gcc gct ggc ggc gta gca act cca gct gat gct gct ctc atg     672
   Asn Phe Ala Ala Gly Gly Val Ala Thr Pro Ala Asp Ala Ala Leu Met
       210                 215                 220
   atg cag ctt ggt gct gac gga gta ttt gtt ggt tct ggt att ttt aaa     720
   Met Gln Leu Gly Ala Asp Gly Val Phe Val Gly Ser Gly Ile Phe Lys
   225                 230                 235                 240
   tca gac aac cct gct aaa ttt gcg aaa gca att gtg gaa gca aca act     768
   Ser Asp Asn Pro Ala Lys Phe Ala Lys Ala Ile Val Glu Ala Thr Thr
                   245                 250                 255
   cac ttt act gat tac aaa tta atc gct gag ttg tca aaa gag ctt ggt     816
   His Phe Thr Asp Tyr Lys Leu Ile Ala Glu Leu Ser Lys Glu Leu Gly
               260                 265                 270
   act gca atg aaa ggg att gaa atc tca aac tta ctt cca gaa cag cgt         864
   Thr Ala Met Lys Gly Ile Glu Ile Ser Asn Leu Leu Pro Glu Gln Arg
           275                 280                 285
   atg caa gaa cgc ggc tgg aga tct att gaa ggc cgc atg aag ttc tcc         912
   Met Gln Glu Arg Gly Trp Arg Ser Ile Glu Gly Arg Met Lys Phe Ser
       290                 295                 300
   att gct gcc gct gtc gtt gct ttc gcc gcc tcc gtc gcg gcc ctc cct         960
   Ile Ala Ala Ala Val Val Ala Phe Ala Ala Ser Val Ala Ala Leu Pro
   305                 310                 315                 320
   cct gcc cat gat tcc cag ttc gct ggc aat ggt gtt ggc aac aag ggc        1008
   Pro Ala His Asp Ser Gln Phe Ala Gly Asn Gly Val Gly Asn Lys Gly
                   325                 330                 335
   aac agc aac gtc aag ttc cct gtc ccc gaa aac gtg acc gtc aag cag        1056
   Asn Ser Asn Val Lys Phe Pro Val Pro Glu Asn Val Thr Val Lys Gln
               340                 345                 350
   gcc tcc gac aag tgc ggt gac cag gcc cag ctc tct tgc tgc aac aag        1104
   Ala Ser Asp Lys Cys Gly Asp Gln Ala Gln Leu Ser Cys Cys Asn Lys
           355                 360                 365
   gcc acg tac gcc ggt gac acc aca acc gtt gat gag ggt ctt ctg tct        1152
   Ala Thr Tyr Ala Gly Asp Thr Thr Thr Val Asp Glu Gly Leu Leu Ser
       370                 375                 380
   ggt gcc ctc agc ggc ctc atc ggc gcc ggg tct ggt gcc gaa ggt ctt        1200
   Gly Ala Leu Ser Gly Leu Ile Gly Ala Gly Ser Gly Ala Glu Gly Leu
   385                 390                 395                 400
   ggt ctc ttc gat cag tgc tcc aag ctt gat gtt gct gtc ctc att ggc        1248
   Gly Leu Phe Asp Gln Cys Ser Lys Leu Asp Val Ala Val Leu Ile Gly
                   405                 410                 415
   atc caa gat ctt gtc aac cag aag tgc aag caa aac att gcc tgc tgc        1296
   Ile Gln Asp Leu Val Asn Gln Lys Cys Lys Gln Asn Ile Ala Cys Cys
               420                 425                 430
   cag aac tcc ccc tcc agc gcg gat ggc aac ctt att ggt gtc ggt ctc        1344
   Gln Asn Ser Pro Ser Ser Ala Asp Gly Asn Leu Ile Gly Val Gly Leu
           435                 440                 445
   cct tgc gtt gcc ctt ggc tcc atc ctc gga tct cat cac cat cac cat        1392
   Pro Cys Val Ala Leu Gly Ser Ile Leu Gly Ser His His His His His
       450                 455                 460
   cac                                                                    1395
   His
   465
<210> 22
   <211> 465
   <212> PRT
   <213> basf-yaad-Xa-rodA-his
<400> 22
   Met Ala Gln Thr Gly Thr Glu Arg Val Lys Arg Gly Met Ala Glu Met
   1               5                   10                  15
   Gln Lys Gly Gly Val Ile Met Asp Val Ile Asn Ala Glu Gln Ala Lys
               20                  25                  30
   Ile Ala Glu Glu Ala Gly Ala Val Ala Val Met Ala Leu Glu Arg Val
           35                  40                  45
   Pro Ala Asp Ile Arg Ala Ala Gly Gly Val Ala Arg Met Ala Asp Pro
       50                  55                  60
   Thr Ile Val Glu Glu Val Met Asn Ala Val Ser Ile Pro Val Met Ala
   65                  70                  75                  80
   Lys Ala Arg Ile Gly His Ile Val Glu Ala Arg Val Leu Glu Ala Met
                   85                  90                  95
   Gly Val Asp Tyr Ile Asp Glu Ser Glu Val Leu Thr Pro Ala Asp Glu
               100                 105                 110
   Glu Phe His Leu Asn Lys Asn Glu Tyr Thr Val Pro Phe Val Cys Gly
           115                 120                 125
   Cys Arg Asp Leu Gly Glu Ala Thr Arg Arg Ile Ala Glu Gly Ala Ser
       130                 135                 140
   Met Leu Arg Thr Lys Gly Glu Pro Gly Thr Gly Asn Ile Val Glu Ala
   145                 150                 155                 160
   Val Arg His Met Arg Lys Val Asn Ala Gln Val Arg Lys Val Val Ala
                   165                 170                 175
   Met Ser Glu Asp Glu Leu Met Thr Glu Ala Lys Asn Leu Gly Ala Pro
               180                 185                 190
   Tyr Glu Leu Leu Leu Gln Ile Lys Lys Asp Gly Lys Leu Pro Val Val
           195                 200                 205
   Asn Phe Ala Ala Gly Gly Val Ala Thr Pro Ala Asp Ala Ala Leu Met
       210                 215                 220
   Met Gln Leu Gly Ala Asp Gly Val Phe Val Gly Ser Gly Ile Phe Lys
   225                 230                 235                 240
   Ser Asp Asn Pro Ala Lys Phe Ala Lys Ala Ile Val Glu Ala Thr Thr
                   245                 250                 255
   His Phe Thr Asp Tyr Lys Leu Ile Ala Glu Leu Ser Lys Glu Leu Gly
               260                 265                 270
   Thr Ala Met Lys Gly Ile Glu Ile Ser Asn Leu Leu Pro Glu Gln Arg
           275                 280                 285
   Met Gln Glu Arg Gly Trp Arg Ser Ile Glu Gly Arg Met Lys Phe Ser
       290                 295                 300
   Ile Ala Ala Ala Val Val Ala Phe Ala Ala Ser Val Ala Ala Leu Pro
   305                 310                 315                 320
   Pro Ala His Asp Ser Gln Phe Ala Gly Asn Gly Val Gly Asn Lys Gly
                   325                 330                 335
   Asn Ser Asn Val Lys Phe Pro Val Pro Glu Asn Val Thr Val Lys Gln
               340                 345                 350
   Ala Ser Asp Lys Cys Gly Asp Gln Ala Gln Leu Ser Cys Cys Asn Lys
           355                 360                 365
   Ala Thr Tyr Ala Gly Asp Thr Thr Thr Val Asp Glu Gly Leu Leu Ser
       370                 375                 380
   Gly Ala Leu Ser Gly Leu Ile Gly Ala Gly Ser Gly Ala Glu Gly Leu
   385                 390                 395                 400
   Gly Leu Phe Asp Gln Cys Ser Lys Leu Asp Val Ala Val Leu Ile Gly
                   405                 410                 415
   Ile Gln Asp Leu Val Asn Gln Lys Cys Lys Gln Asn Ile Ala Cys Cys
               420                 425                 430
   Gln Asn Ser Pro Ser Ser Ala Asp Gly Asn Leu Ile Gly Val Gly Leu
           435                 440                 445
   Pro Cys Val Ala Leu Gly Ser Ile Leu Gly Ser His His His His His
       450                 455                 460
   His
   465
<210> 23
   <211> 1407
   <212> DNA
   <213> basf-yaad-Xa-BASF1-his
<220>
   <221> CDS
   <222> (1)..(1407)
   <223>
<400> 23
   atg gct caa aca ggt act gaa cgt gta aaa cgc gga atg gca gaa atg       48
   Met Ala Gln Thr Gly Thr Glu Arg Val Lys Arg Gly Met Ala Glu Met
   1               5                   10                  15
   caa aaa ggc ggc gtc atc atg gac gtc atc aat gcg gaa caa gcg aaa       96
   Gln Lys Gly Gly Val Ile Met Asp Val Ile Asn Ala Glu Gln Ala Lys
               20                  25                  30
   atc gct gaa gaa gct gga gct gtc gct gta atg gcg cta gaa cgt gtg      144
   Ile Ala Glu Glu Ala Gly Ala Val Ala Val Met Ala Leu Glu Arg Val
           35                  40                  45
   cca gca gat att cgc gcg gct gga gga gtt gcc cgt atg gct gac cct      192
   Pro Ala Asp Ile Arg Ala Ala Gly Gly Val Ala Arg Met Ala Asp Pro
       50                  55                  60
   aca atc gtg gaa gaa gta atg aat gca gta tct atc ccg gta atg gca      240
   Thr Ile Val Glu Glu Val Met Asn Ala Val Ser Ile Pro Val Met Ala
   65                  70                  75                  80
   aaa gcg cgt atc gga cat att gtt gaa gcg cgt gtg ctt gaa gct atg      288
   Lys Ala Arg Ile Gly His Ile Val Glu Ala Arg Val Leu Glu Ala Met
                   85                  90                  95
   ggt gtt gac tat att gat gaa agt gaa gtt ctg acg ccg gct gac gaa      336
   Gly Val Asp Tyr Ile Asp Glu Ser Glu Val Leu Thr Pro Ala Asp Glu
               100                 105                 110
   gaa ttt cat tta aat aaa aat gaa tac aca gtt cct ttt gtc tgt ggc      384
   Glu Phe His Leu Asn Lys Asn Glu Tyr Thr Val Pro Phe Val Cys Gly
           115                 120                 125
   tgc cgt gat ctt ggt gaa gca aca cgc cgt att gcg gaa ggt gct tct      432
   Cys Arg Asp Leu Gly Glu Ala Thr Arg Arg Ile Ala Glu Gly Ala Ser
       130                 135                 140
   atg ctt cgc aca aaa ggt gag cct gga aca ggt aat att gtt gag gct      480
   Met Leu Arg Thr Lys Gly Glu Pro Gly Thr Gly Asn Ile Val Glu Ala
   145                 150                 155                 160
   gtt cgc cat atg cgt aaa gtt aac gct caa gtg cgc aaa gta gtt gcg      528
   Val Arg His Met Arg Lys Val Asn Ala Gln Val Arg Lys Val Val Ala
                   165                 170                 175
   atg agt gag gat gag cta atg aca gaa gcg aaa aac cta ggt gct cct      576
   Met Ser Glu Asp Glu Leu Met Thr Glu Ala Lys Asn Leu Gly Ala Pro
               180                 185                 190
   tac gag ctt ctt ctt caa att aaa aaa gac ggc aag ctt cct gtc gtt      624
   Tyr Glu Leu Leu Leu Gln Ile Lys Lys Asp Gly Lys Leu Pro Val Val
           195                 200                 205
   aac ttt gcc gct ggc ggc gta gca act cca gct gat gct gct ctc atg      672
   Asn Phe Ala Ala Gly Gly Val Ala Thr Pro Ala Asp Ala Ala Leu Met
       210                 215                 220
   atg cag ctt ggt gct gac gga gta ttt gtt ggt tct ggt att ttt aaa      720
   Met Gln Leu Gly Ala Asp Gly Val Phe Val Gly Ser Gly Ile Phe Lys
   225                 230                 235                 240
   tca gac aac cct gct aaa ttt gcg aaa gca att gtg gaa gca aca act      768
   Ser Asp Asn Pro Ala Lys Phe Ala Lys Ala Ile Val Glu Ala Thr Thr
                   245                 250                 255
   cac ttt act gat tac aaa tta atc gct gag ttg tca aaa gag ctt ggt      816
   His Phe Thr Asp Tyr Lys Leu Ile Ala Glu Leu Ser Lys Glu Leu Gly
               260                 265                 270
   act gca atg aaa ggg att gaa atc tca aac tta ctt cca gaa cag cgt      864
   Thr Ala Met Lys Gly Ile Glu Ile Ser Asn Leu Leu Pro Glu Gln Arg
           275                 280                 285
   atg caa gaa cgc ggc tgg aga tct att gaa ggc cgc atg aag ttc tcc      912
   Met Gln Glu Arg Gly Trp Arg Ser Ile Glu Gly Arg Met Lys Phe Ser
       290                 295                 300
   gtc tcc gcc gcc gtc ctc gcc ttc gcc gcc tcc gtc gcc gcc ctc cct      960
   Val Ser Ala Ala Val Leu Ala Phe Ala Ala Ser Val Ala Ala Leu Pro
   305                 310                 315                 320
   cag cac gac tcc gcc gcc ggc aac ggc aac ggc gtc ggc aac aag ttc     1008
   Gln His Asp Ser Ala Ala Gly Asn Gly Asn Gly Val Gly Asn Lys Phe
                   325                 330                 335
   cct gtc cct gac gac gtc acc gtc aag cag gcc acc gac aag tgc ggc     1056
   Pro Val Pro Asp Asp Val Thr Val Lys Gln Ala Thr Asp Lys Cys Gly
               340                 345                 350
   gac cag gcc cag ctc tcc tgc tgc aac aag gcc acc tac gcc ggc gac     1104
   Asp Gln Ala Gln Leu Ser Cys Cys Asn Lys Ala Thr Tyr Ala Gly Asp
           355                 360                 365
   gtc ctc acc gac atc gac gag ggc atc ctc gcc ggc ctc ctc aag aac     1152
   Val Leu Thr Asp Ile Asp Glu Gly Ile Leu Ala Gly Leu Leu Lys Asn
       370                 375                 380
   ctc atc ggc ggc ggc tcc ggc tcc gag ggc ctc ggc ctc ttc gac cag     1200
   Leu Ile Gly Gly Gly Ser Gly Ser Glu Gly Leu Gly Leu Phe Asp Gln
   385                 390                 395                 400
   tgc gtc aag ctc gac ctc cag atc tcc gtc atc ggc atc cct atc cag     1248
   Cys Val Lys Leu Asp Leu Gln Ile Ser Val Ile Gly Ile Pro Ile Gln
                   405                 410                 415
   gac ctc ctc aac cag gtc aac aag cag tgc aag cag aac atc gcc tgc     1296
   Asp Leu Leu Asn Gln Val Asn Lys Gln Cys Lys Gln Asn Ile Ala Cys
               420                 425                 430
   tgc cag aac tcc cct tcc gac gcc acc ggc tcc ctc gtc aac ctc ggc     1344
   Cys Gln Asn Ser Pro Ser Asp Ala Thr Gly Ser Leu Val Asn Leu Gly
           435                 440                 445
   ctc ggc aac cct tgc atc cct gtc tcc ctc ctc cat atg gga tct cat     1392
   Leu Gly Asn Pro Cys Ile Pro Val Ser Leu Leu His Met Gly Ser His
       450                 455                 460
   cac cat cac cat cac                                                 1407
   His His His His His
   465
<210> 24
   <211> 469
   <212> PRT
   <213> basf-yaad-Xa-BASF1-his
<400> 24
   Met Ala Gln Thr Gly Thr Glu Arg Val Lys Arg Gly Met Ala Glu Met
   1 5 10 15
   Gln Lys Gly Gly Val Ile Met Asp Val Ile Asn Ala Glu Gln Ala Lys
   20 25 30
   Ile Ala Glu Glu Ala Gly Ala Val Ala Val Met Ala Leu Glu Arg Val
   35 40 45
   Pro Ala Asp Ile Arg Ala Ala Gly Gly Val Ala Arg Met Ala Asp Pro
   50 55 60
   Thr Ile Val Glu Glu Val Met Asn Ala Val Ser Ile Pro Val Met Ala
   65 70 75 80
   Lys Ala Arg Ile Gly His Ile Val Glu Ala Arg Val Leu Glu Ala Met
   85 90 95
   Gly Val Asp Tyr Ile Asp Glu Ser Glu Val Leu Thr Pro Ala Asp Glu
   100 105 110
   Glu Phe His Leu Asn Lys Asn Glu Tyr Thr Val Pro Phe Val Cys Gly
   115 120 125
   Cys Arg Asp Leu Gly Glu Ala Thr Arg Arg Ile Ala Glu Gly Ala Ser
   130 135 140
   Met Leu Arg Thr Lys Gly Glu Pro Gly Thr Gly Asn Ile Val Glu Ala
   145 150 155 160
   Val Arg His Met Arg Lys Val Asn Ala Gln Val Arg Lys Val Val Ala
   165 170 175
   Met Ser Glu Asp Glu Leu Met Thr Glu Ala Lys Asn Leu Gly Ala Pro
   180 185 190
   Tyr Glu Leu Leu Leu Gln Ile Lys Lys Asp Gly Lys Leu Pro Val Val
           195                 200                 205
   Asn Phe Ala Ala Gly Gly Val Ala Thr Pro Ala Asp Ala Ala Leu Met
       210                 215                 220
   Met Gln Leu Gly Ala Asp Gly Val Phe Val Gly Ser Gly Ile Phe Lys
   225                 230                 235                 240
   Ser Asp Asn Pro Ala Lys Phe Ala Lys Ala Ile Val Glu Ala Thr Thr
                   245                 250                 255
   His Phe Thr Asp Tyr Lys Leu Ile Ala Glu Leu Ser Lys Glu Leu Gly
               260                 265                 270
   Thr Ala Met Lys Gly Ile Glu Ile Ser Asn Leu Leu Pro Glu Gln Arg
           275                 280                 285
   Met Gln Glu Arg Gly Trp Arg Ser Ile Glu Gly Arg Met Lys Phe Ser
       290                 295                 300
   Val Ser Ala Ala Val Leu Ala Phe Ala Ala Ser Val Ala Ala Leu Pro
   305                 310                 315                 320
   Gln His Asp Ser Ala Ala Gly Asn Gly Asn Gly Val Gly Asn Lys Phe
                   325                 330                 335
   Pro Val Pro Asp Asp Val Thr Val Lys Gln Ala Thr Asp Lys Cys Gly
               340                 345                 350
   Asp Gln Ala Gln Leu Ser Cys Cys Asn Lys Ala Thr Tyr Ala Gly Asp
           355                 360                 365
   Val Leu Thr Asp Ile Asp Glu Gly Ile Leu Ala Gly Leu Leu Lys Asn
       370                 375                 380
   Leu Ile Gly Gly Gly Ser Gly Ser Glu Gly Leu Gly Leu Phe Asp Gln
   385                 390                 395                 400
   Cys Val Lys Leu Asp Leu Gln Ile Ser Val Ile Gly Ile Pro Ile Gln
                   405                 410                 415
   Asp Leu Leu Asn Gln Val Asn Lys Gln Cys Lys Gln Asn Ile Ala Cys
               420                 425                 430
   Cys Gln Asn Ser Pro Ser Asp Ala Thr Gly Ser Leu Val Asn Leu Gly
           435                 440                 445
   Leu Gly Asn Pro Cys Ile Pro Val Ser Leu Leu His Met Gly Ser His
       450                 455                 460
   His His His His His
   465

## Beschreibung

Die vorliegende Erfindung betrifft eine Bohrspülung enthaltend ein Hydrophobin oder eines seiner Derivate sowie die Verwendung eines Hydrophobins als Hilfsmittel in einer Bohrspülung.

Bohrspülungen haben im Allgemeinen die Aufgabe, bei der Erschließung neuer Lagerstätten für beispielsweise Erdöl oder Erdgas den oftmals schwierigen Bohrvorgang zu vereinfachen. Dabei soll sowohl der Bohrvorgang selbst wie auch die Förderung der dabei erzeugten Gesteinssplitter unterstützt werden. Der Bohrmeißel und das Bohrgestänge sind zu schmieren und zu kühlen. Ferner ist der hydrostatische Druck der Lagerstätte zu kompensieren, weshalb häufig Bohrspülungen mit einem erhöhten spezifischen Gewicht zum Einsatz gebracht werden. Auch sollten die Wände der Bohrung ausgekleidet werden.

Wichtige Eigenschaften einer kommerziell verwertbaren Bohrspülung sind unter anderem auch geeignete Viskosität und Fließeigenschaften, Dichte, Temperaturstabilität, Emulgier- und Dispergiervermögen, pH-Kontrolle sowie nicht zuletzt auch ein hohes Maß an Umweltfreundlichkeit.

Da die Suche nach neuen Rohstoffen an vielen Stellen der Erde fortgesetzt wird, wobei häufig auch an abgelegenen Stellen, beispielsweise fern der Küste oder mitten im Urwald, tiefe Bohrungen vorgenommen werden müssen, besteht ein Bedarf an neuen Bohrhilfsmitteln, insbesondere Hilfsmitteln für eine Bohrspülung, die den hohen technischen Anforderungen gerecht werden sowie kostengünstig hergestellt und vor Ort eingesetzt werden können. Darüber hinaus solite eine Bohrspülung auch lagerstabil sein und über einen längeren Zeitraum eingesetzt werden können.

Vor besonderer Bedeutung ist es ferner, dass eine Bohrspülung bei einer Freisetzung in der Umwelt nicht zu nachhaltigen Schäden in der Tier- und Pflanzenwelt führt.

Hydrophobine sind kleine Proteine von etwa 100 bis 150 Aminosäuren, die von filamentösen Pilzen, beispielsweise von *Schizophyllum commune,* hergestellt werden können. Sie weisen in aller Regel 8 Cystein-Einheiten pro Molekül auf.

Hydrophobine weisen eine ausgeprägte Affinität zu Grenzflächen auf und eignen sich daher zur Beschichtung von Oberflächen, z. B. um die Eigenschaften der Grenzflächen durch Bildung von amphiphatischen Membranen zu verändern. So lässt sich beispielsweise der Kunststoff Teflon mittels Hydrophobinen unter Erhalt einer hydrophilen Oberfläche beschichten.

Hydrophobine können aus natürlichen Quellen isoliert werden. Ferner sind synthetische Herstellverfahren für Hydrophobine und ihre Derivate bekannt. Beispielsweise offenbart die deutsche Patentanmeldung DE 10 2005 007 480.4 ein Herstellverfahren für Hydrophobine und Derivate davon.

Im Stand der Technik ist die Verwendung von Hydrophobinen für verschiedene Anwendungen bereits vorgeschlagen worden.

So wird in EP-A 05 016 962 die Verwendung von Proteinen zur Verbesserung der Phasentrennung von z.B. Öl/Wasser- oder Kraftstoff/Wassergemischen beschrieben. Dem Fachmann ist bekannt, dass bestimmte amphiphile Moleküle je nach Anwendungskonzentration und umgebendem Medium sowohl stabilisierend als auch destabilisierend auf Phasengrenzen wirken können.

WO 96/41882 schlägt die Verwendung von Hydrophobinen als Emulgatoren, Verdicker, oberflächenaktive Substanzen, zum Hydrophilieren hydrophober Oberflächen, zur Verbesserung der Wasserbeständigkeit hydrophiler Substrate, zur Herstellung von Öl-in-Wasser-Emulsionen oder von Wasser-in-Öl-Emulsionen vor. Weiterhin werden pharmazeutische Anwendungen wie die Herstellung von Salben oder Cremes sowie kosmetische Anwendungen wie Hautschutz oder die Herstellung von Haarshampoos oder Haarspülungen vorgeschlagen. WO 96/41882 beschreibt darüber hinaus Zusammensetzungen z. B. für pharmazeutische Anwendungen enthaltend ein Hydrophobin.

EP-A 1 252 516 offenbart die Beschichtung von Fenstern, Kontaktlinsen, Biosensoren, medizinischen Vorrichtungen, Behältern zur Durchführung von Versuchen oder zur Lagerung, Schiffrümpfen, festen Teilchen oder Rahmen oder Karosserie von Personenkraftwagen mit einer Hydrophobin enthaltenden Lösung bei einer Temperatur von 30 bis 80°C.

WO 03/53383 beschreibt die Verwendung von Hydrophobin zum Behandeln von Keratin-Materialien in kosmetischen Anwendungen.

WO 03/10331 offenbart, dass Hydrophobine oberflächenaktive Eigenschaften aufweisen. So wird ein mit Hydrophobin beschichteter Sensor offenbart, beispielsweise eine Messelektrode, an den nicht kovalent weitere Substanzen, z.B. elektroaktive Substanzen, Antikörper oder Enzyme gebunden sind.

WO 2004/000880 stellt die Beschichtung von Oberflächen mit Hydrophobin oder Hydrophobin-ähnlichen Substanzen vor. Weiter wird offenbart, dass auch Öl-in-Wasser oder Wasser-in-Öl-Emulsionen durch Zugabe von Hydrophobinen stabilisiert werden können.

Auch WO 01/74864, die Hydrophobin-ähnliche Proteine betrifft, beschreibt, dass diese zur Stabilisierung von Dispersionen und Emulsionen eingesetzt werden können.

Auch ist die Verwendung von Proteinen zur Phasentrennung prinzipiell bekannt. So beschreibt GB 195,876 ein Verfahren zum Brechen von Wasser-in-Öl-Emulsionen unter Verwendung von Kolloiden. Als Kolloide werden beispielhaft Proteine wie Gelatine, Casein, Albumin oder Polysccharide wie Gummi Arabicum oder Gummi Tragacanth genannt.

JP-A 11-169177 offenbart die Verwendung von Proteinen mit Lipase-Aktivität zum Brechen von Emulsionen.

WO 01/60916 offenbart die Verwendung von tensidfreien Mischungen aus mindestens einem wasserlöslichen Protein, mindestens einem wasserlöslichen Polysaccharid sowie mindestens einem wasserlöslichen Polymer wie bspw. Polyethylenoxid für verschiedene Anwendungen, darunter auch zum Demulgieren von Rohöl.

Das Dokument GB-A 2 235 457 beschreibt ein Verfahren zur Gewinnung eines Proteins mit Tensidwirkung aus *Ochrobactum* sowie dessen Einsatz unter anderem in Bohrspülungen.

Keine der zitierten Schriften offenbart jedoch die Verwendung von Hydrophobinen als Hilfsmittel in Bohrspülungen.

Die vorliegende Erfindung hat die Verwendung eines Hydrophobins als Hilfsmittel in einer Bohrspülung zum Gegenstand, wobei das Hydrophobin insbesondere als Emulgator in einer Bohrspülung eingesetzt wird.

Bevorzugterweise ist dabei das eingesetzte Hydrophobin ein Fusions-Hydrophobin, insbesondere ein Fusions-Hydrophobin ausgewählt aus der später erläuterten Gruppe von yaad-Xa-dewA-his (SEQ ID NO: 20), yaad-Xa-rodA-his (SEQ ID NO: 22) oder yaad-Xa-basf1-his (SEQ ID NO: 24), wobei es sich bei yaad auch um einen verkürzten Fusionspartner yaad' mit 20 bis 293 Aminosäuren handeln kann.

Bohrspülungen bestehen in der Regel aus einer oder mehreren Flüssigkeiten wie z.B. Wasser, Rohöl und organischen Additiven bzw. Lösungsmitteln sowie aus suspendierten oder gelösten Feststoffen. Je nach Art der Haupt-Flüssigkeitskomponente unterscheidet man zwischen wässrigen Bohrspülungen, Öl-basierten Bohrspülungen und synthetischen Bohrspülungen.

Bei der erfindungsgemäßen Bohrspülung wird bevorzugt eine Bohrspülung auf Öl-Basis (z.B. Diesel, Mineral-Öl) eingesetzt. Ebenfalls bevorzugt wird das Hydrophobin (es können auch mehrere Hydrophobine gleichzeitig eingesetzt werden) in einer Menge von 0,1 bis 10.000 ppm, vorzugsweise 1 bis 1000 ppm, insbesondere 1 bis 600 ppm, jeweils bezogen auf die gesamte Zusammensetzung, eingesetzt. Es ist einer der Vorzüge, dass die eingesetzte Menge an Hydrophobin deutlich kleiner ist als bei herkömmlichen Emulgatoren, die im Bereich von 2 bis 10 Gew.-% eingesetzt werden.

Vorzugsweise handelt es sich um eine Bohrspülung auf Öl-Basis, die 40 bis 95 , vorzugsweise 70 bis 95 Gew.-% mindestens einer Ölkomponente, 2 bis 80, vorzugsweise 2 bis 30 Gew.-% Wasser sowie gegebenenfalls weitere Komponenten enthält.

Als weitere Komponenten kommen z. B. folgende Substanzen in Frage:
a) Salze (wie z.B. Alkali- und Erdalkalichloride, -bromide, -carbonate; diese Salze dienen oftmals auch der pH-Kontrolle),
b) Additive zum Ausgleich von Flüssigkeitsverlusten (z. B. Bentonite, Stärke, Cellulose oder Derivate davon),
c) Benetzungsmittel [um die o.g. Additive (z.B. Bentonite) ölbenetzbar zu machen],
d) Fließverbesserer (die den Fließwiderstand vermindern, beispielsweise Acrylharze, Polysiloxane, Polyurethane),
e) Additive zur Erhöhung des spezifischen Gewichts der Bohrspülung (z. B. Barit, Hematit, Magnetit, Ilmenit, Siderit, Dolomit, Calzit, Kochsalz),
f) Emulgatoren (z.B. Salze von Fettsäuren; Polyamide),
g) sonstige Zusätze wie z.B. Dispergiermittel, Fluid Loss Additive, Polymere oder Copolymere, Amine (z.B. Polyacrylamide)
h) Viskositäts-erhöhende Zusätze (z.B. Bentonit, Attapulgit).

In einer bevorzugten Ausführungsform der Erfindung kommt neben dem Hydrophobin mindestens eine weitere Verbindung zum Einsatz, die die Emulsionsbildung verbessert.

Die Erfindung betrifft auch ein Verfahren zur Bohrung eines Bohrloches, insbesondere zur Erschließung von unterirdischen Lagerstätten, insbesondere von Öl und Gas, bei dem eine Bohrspülung eingesetzt wird, die mindestens ein Hydrophobin oder ein Derivat desselben enthält. Dabei werden die oben genannten Fusions-Hydrophobine bevorzugt eingesetzt.

Bei dem Bohrverfahren wird zumeist eine Bohrspülung auf Öl-Basis eingesetzt, die 40 bis 95 Gew.-% mindestens einer Ölkomponente wie z. B. Biodiesel, 2 bis 60 Gew.-% Wasser (z. B. Süß- oder Meerwasser) sowie gegebenenfalls weitere Komponenten enthält.

Ferner ist auch eine Bohrspülung selbst, enthaltend mindestens ein Hydrophobin, Gegenstand der vorliegenden Erfindung.

In einer besonderen Ausführungsform enthält die Bohrspülung 70 bis 95 Gew.-% mindestens einer Ölkomponente (z. B. Biodiesel), 2 bis 30 Gew.-% Wasser, sowie gegebenenfalls bis zu 13 Gew.-% weiterer Komponenten (z.B. Bentonit und/oder Calciumchlorid).

Die Erfindung betrifft auch ein Verfahren zur Herstellung einer Bohrspülung, bei dem die Hydrophobin-Komponente und die übrigen Komponenten der Bohrspülung innig vermischt werden. Dieses kann an einem industriellen Produktionsort geschehen aber auch vor Ort, beispielsweise auf einer Bohrplattform.

Die Verwendung von Proteinen wie Hydrophobin als Bohrhilfsmittel hat den Vorteil, dass es sich um natürlich vorkommende oder natur-analoge Substanzen handelt, die biologisch abbaubar sind und damit nicht zu einer dauerhaften Belastung der Umwelt führen.

Bei vielen Anwendungen im großen Maßstab, beispielsweise bei der Bohrung nach Ölvorkommen, kommt es auf eine möglichst dauerhafte Verwendbarkeit der eingesetzten Hilfsmittel an. Aufgabe der Erfindung war es, ein verbessertes Verfahren zur Vornahme von Erd-Bohrungen unter Einsatz spezieller Proteine bereitzustellen.

Unter einem Hydrophobin werden im Rahmen der vorliegenden Erfindung auch Derivate davon bzw. modifizierte Hydrophobin verstanden. Bei modifizierten bzw. derivatisierten Hydrophobinen kann es sich beispielsweise um Hydrophobinfusionsproteine handeln oder um Proteine, die eine Aminosäurensequenz aufweisen, die mindestens 60%, beispielsweise mindestens 70 %, insbesondere mindestens 80 %, besonders bevorzugt mindestens 90 %, insbesondere bevorzugt mindestens 95 % Identität mit der Sequenz eines Hydrophobins aufweisen, und die noch zu 50 %, beispielsweise zu 60 %, insbesondere zu 70 %, besonders bevorzugt zu 80 %, die biologischen Eigenschaften eines Hydrophobins erfüllen, insbesondere die Eigenschaft, dass die Oberflächeneigenschaften durch Beschichten mit diesen Proteinen derart geändert werden, dass der Kontaktwinkel eines Wassertropfens vor und nach der Beschichtung einer Glasoberfläche mit dem Protein eine Vergrößerung um mindestens 20°, bevorzugt um mindestens 25°, insbesondere um mindestens 30° aufweist.

Überraschenderweise wurde gefunden, dass Hydrophobine oder Derivate davon, die Bohrleistung bei der Erkundung und Erschließung von Lagerstätten verbessern. Dies beruht auch darauf, dass eine schnelle Phasentrennung der Bohrspülung und des Förderguts vermieden wird. Hierbei sind schon geringe Mengen an Hydrophobin äußerst wirksam. wird.

Für die Definition von Hydrophobinen ist dabei die Struktur- und nicht die Sequenzspezifität der Hydrophobine entscheidend. Die Aminosäuresequenz der natürlichen Hydrophobine ist sehr divers, sie haben jedoch alle ein hochcharakteristisches Muster von 8 konservierten Cysteinresten. Diese Reste bilden vier intramolekulare Disulfidbrücken. Der N- und C-Terminus ist über einen größeren Bereich variabel. Hier können mittels dem Fachmann bekannten molekularbiologischen Techniken Fusionspartnerproteine mit einer Länge von 10 bis 500 Aminosäuren angefügt werden. Darüber hinaus sind im Sinne der vorliegenden Erfindung unter Hydrophobinen und Derivaten davon Proteine mit einer ähnlichen Struktur und funktioneller Äquivalenz zu verstehen.

Unter dem Begriff "Hydrophobine" im Sinne der vorliegenden Erfindung sollen im Folgenden Polypeptide der allgemeinen Strukturformel (I)

Xₙ-C¹-X₁₋₅₀-C²-X₀₋₅-C³-X₁₋₁₀₀-C⁴-X₁₋₁₀₀-C⁵-X₁₋₅₀-C⁶-X₀₋₅-C⁷-X₁₋₅₀-C⁸-Xₘ (I)

verstanden werden, wobei X für jede der 20 natürlich vorkommenden Aminosäuren (Phe, Leu, Ser, Tyr, Cys, Trp, Pro, His, Gln, Arg, Ile Met, Thr, Asn, Lys, Val, Ala, Asp, Glu, Gly) stehen kann. Dabei können X jeweils gleich oder verschieden sein. Hierbei stellen die bei X stehenden Indizes jeweils die Anzahl der Aminosäuren dar, C steht für Cystein, Alanin, Serin, Glycin, Methionin oder Threonin, wobei mindestens vier der mit C benannten Reste für Cystein stehen, und die Indizes n und m stehen unabhängig voneinander für natürliche Zahlen zwischen 0 und 500, bevorzugt zwischen 15 und 300.

Die Polypeptide gemäß der Formel (I) sind weiterhin durch die Eigenschaft charakterisiert, dass sie bei Raumtemperatur nach Beschichten einer Glasoberfläche eine Vergrößerung des Kontaktwinkels eines Wassertropfens von mindestens 20°, bevorzugt mindestens 25° und besonders bevorzugt 30° bewirken, jeweils verglichen mit dem Kontaktwinkel eines gleich großen Wassertropfens mit der unbeschichteten Glasoberfläche.

Die mit C¹ bis C⁸ benannten Aminosäuren sind bevorzugt Cysteine; sie können aber auch durch andere Aminosäuren ähnlicher Raumerfüllung, bevorzugt durch Alanin, Serin, Threonin, Methionin oder Glycin ersetzt werden. Allerdings sollen mindestens vier, bevorzugt mindestens 5, besonders bevorzugt mindestens 6 und insbesondere mindestens 7 der Positionen C¹ bis C⁸ aus Cysteinen bestehen. Cysteine können in den erfindungsgemäßen Proteinen entweder reduziert vorliegen oder miteinander Disulfidbrücken ausbilden. Besonders bevorzugt ist die intramolekulare Ausbildung von C-C Brücken, insbesondere die mit mindestens einer, bevorzugt 2, besonders bevorzugt 3 und ganz besonders bevorzugt 4 intramolekularen Disulfidbrücken. Bei dem oben beschriebenen Austausch von Cysteinen durch Aminosäuren ähnlicher Raumerfüllung werden vorteilhaft solche C-Positionen paarweise ausgetauscht, die intramolekulare Disulfidbrücken untereinander ausbilden können.

Falls in den mit X bezeichneten Positionen auch Cysteine, Serine, Alanine, Glycine, Methionine oder Threonine verwendet werden, kann sich die Nummerierung der einzelnen C-Positionen in den allgemeinen Formeln entsprechend verändern.

Bevorzugt werden Hydrophobine der allgemeinen Formel (II)

Xₙ-C¹ -X₃₋₂₅-C² -X₀₋₂-C³ -X₅₋₅₀-C⁴ -X₂₋₃₅-C⁵-X₂₋₁₅-C⁶ -X₀₋₂-C⁷ -X₃₋₃₅-C⁸-Xₘ (II)

zur Ausführung der vorliegenden Erfindung eingesetzt, wobei X, C und die bei X und C stehenden Indizes die obige Bedeutung haben, die Indizes n und m für Zahlen zwischen 0 und 300 stehen, und sich die Proteine weiterhin durch die oben erwähnte Kontaktwinkeländerung auszeichnen, und es sich weiterhin bei mindestens 6 der mit C benannten Reste um Cystein handelt. Besonders bevorzugt handelt es sich bei allen Reste C um Cystein.

Besonders bevorzugt werden Hydrophobine der allgemeinen Formel (III)

Xₙ-C¹-X₅₋₉-C²-C³-X₁₁₋₃₉-C⁴-X₂₋₂₃-C⁵-X₅₋₉-C⁶-C⁷-C⁷-C₆₋₈-C⁸-Xₘ (III)

eingesetzt, wobei X, C und die bei X stehenden Indizes die obige Bedeutung haben, die Indizes n und m für Zahlen zwischen 0 und 200 stehen, sich die Proteine weiterhin durch die oben erwähnte Kontaktwinkeländerung auszeichnen, und es sich bei mindestens 6 der mit C benannten Reste um Cystein handelt. Besonders bevorzugt handelt es sich bei allen Resten C um Cystein.

Bei den Resten Xₙ und Xₘ kann es sich um Peptidsequenzen handeln, die natürlicherweise auch mit einem Hydrophobin verknüpft sind. Es kann sich aber auch bei einem oder beiden Resten um Peptidsequenzen handeln, die natürlicherweise nicht mit einem Hydrophobin verknüpft sind. Darunter sind auch solche Reste Xₙ und/oder Xₘ zu verstehen, bei denen eine natürlicherweise in einem Hydrophobin vorkommende Peptidsequenz durch eine nicht natürlicherweise in einem Hydrophobin vorkommende Peptidsequenz verlängert ist.

Falls es sich bei Xₙ und/oder Xₘ um natürlicherweise nicht in Hydrophobinen verknüpfte Peptidsequenzen handelt, sind derartige Sequenzen in der Regel mindestens 20, bevorzugt mindestens 35, besonders bevorzugt mindestens 50 und ganz besonders bevorzugt mindestens 100 Aminosäuren lang. Ein derartiger, natürlicherweise nicht mit einem Hydrophobin verknüpfter Rest soll im Folgenden auch als Fusionspartner bezeichnet werden. Damit soll ausgedrückt werden, dass die Proteine aus mindestens einem Hydrophobinteil und einem Fusionspartnerteil bestehen können, die in der Natur nicht zusammen in dieser Form vorkommen.

Der Fusionspartnerteil kann aus einer Vielzahl von Proteinen ausgewählt werden. Es können auch mehrere Fusionspartner mit einem Hydrophobinteil verknüpft werden, beispielsweise am Aminoterminus (Xₙ) und am Carboxyterminus (Xₘ) des Hydrophobinteils. Es können aber auch beispielsweise zwei Fusionspartner mit einer Position (Xₙ oder Xₘ) des erfindungsgemäßen Proteins verknüpft werden.

Besonders geeignete Fusionspartner sind Proteine, die natürlicherweise in Mikroorganismen, insbesondere in E. coli oder Bacillus subtilis vorkommen. Beispiele für solche Fusionspartner sind die Sequenzen yaad (SEQ ID NO: 15 und 16), yaae (SEQ ID NO: 17 und 18), und Thioredoxin.

Gut geeignet sind auch Fragmente oder Derivate dieser genannten Sequenzen, die nur einen Teil, beispielsweise 70 bis 99 %, bevorzugt 5 bis 50 %, und besonders bevorzugt 10 bis 40 % der genannten Sequenzen umfassen, oder bei denen einzelne Aminosäuren, beziehungsweise Nukleotide gegenüber der genannten Sequenz verändert sind, wobei sich die Prozentangaben jeweils auf die Anzahl der Aminosäuren bezieht.

In einer weiterhin bevorzugten Ausführungsform weist das Fusion-Hydrophobin neben dem Fusionspartner als eine Gruppe Xₙ oder Xₘ noch eine sogenannte Affinitätsdomäne (affinity tag / affinity tail) auf. Hierbei handelt es sich in prinzipiell bekannter Art und Weise um Ankergruppen, welche mit bestimmten komplementären Gruppen wechselwirken können und der leichteren Aufarbeitung und Reinigung der Proteine dienen können. Beispiele derartiger Affinitätsdomänen umfassen (His)ₖ- , (Arg)ₖ-, (Asp)ₖ-, (Phe)ₖ- oder (Cys)ₖ-Gruppen, wobei k im allgemeinen für eine natürliche Zahl von 1 bis 10 steht. Bevorzugt kann es sich um eine (His)ₖ-Gruppe handeln, wobei k für 4 bis 6 steht.

Die erfindungsgemäß als Hydrophobine oder Derivate davon verwendeten Proteine können auch noch in ihrer Polypeptidsequenz modifiziert sein, beispielsweise durch Glycosilierung, Acetylierung oder auch durch chemische Quervernetzung beispielsweise mit Glutardialdehyd.

Eine Eigenschaft der erfindungsgemäß verwendeten Hydrophobine bzw. deren Derivate ist die Änderung von Oberflächeneigenschaften, wenn die Oberflächen mit den Proteinen beschichtet werden. Die Änderung der Oberflächeneigenschaften lässt sich experimentell beispielsweise dadurch bestimmen, dass der Kontaktwinkel eines Wassertropfens vor und nach der Beschichtung der Oberfläche mit dem Protein gemessen wird und die Differenz der beiden Messungen ermittelt wird.

Die Durchführung von Kontaktwinkelmessungen ist dem Fachmann prinzipiell bekannt. Die Messungen beziehen sich auf Raumtemperatur sowie Wassertropfen von 5 µl und die Verwendung von Glasplättchen als Substrat. Die genauen experimentellen Bedingungen für eine beispielhaft geeignete Methode zur Messung des Kontaktwinkels sind im experimentellen Teil dargestellt. Unter den dort genannten Bedingungen besitzen die erfindungsgemäß verwendeten Fusionsproteine die Eigenschaft, den Kontaktwinkel um mindestens 20°, bevorzugt mindestens 25°, besonders bevorzugt mindestens 30° zu vergrößern, jeweils verglichen mit dem Kontaktwinkel eines gleich großen Wassertropfens mit der unbeschichteten Glasoberfläche.

Besonders bevorzugte Hydrophobine zur Ausführung der vorliegenden Erfindung sind die Hydrophobine des Typs dewA, rodA, hypA, hypB, sc3, basf1, basf2, die im nachfolgenden Sequenzprotokoll strukturell charakterisiert sind. Es kann sich auch nur um Teile oder Derivate davon handeln. Es können auch mehrere Hydrophobinteile, bevorzugt 2 oder 3, gleicher oder unterschiedlicher Struktur miteinander verknüpft und mit einer entsprechenden geeigneten Polypeptidsequenz, die natürlicherweise nicht mit einem Hydrophobin verbunden ist, verknüpft werden.

Erfindungsgemäß besonders geeignet sind weiterhin die Fusionsproteine yaad-Xa-dewA-his (SEQ ID NO: 20), yaad-Xa-rodA-his (SEQ ID NO: 22) oder yaad-Xa-basf1-his (SEQ ID NO: 24) mit den in Klammern angegebenen Polypeptidsequenzen sowie den dafür codierenden Nukleinsäuresequenzen, insbesondere den Sequenzen gemäß SEQ ID NO: 19, 21, 23. Auch Proteine, die sich ausgehend von den in SEQ ID NO. 20, 22 oder 24 dargestellten Polypeptidsequenzen durch Austausch, Insertion oder Deletion von mindestens einer, bis hin zu 10, bevorzugt 5, besonders bevorzugt 5% aller Aminosäuren ergeben, und die die biologische Eigenschaft der Ausgangsproteine noch zu mindestens 50% besitzen, sind besonders bevorzugte Ausführungsformen. Unter biologischer Eigenschaft der Proteine wird hierbei die bereits beschriebene Änderung des Kontaktwinkels um mindestens 20° verstanden.

Besonders zur Ausführung der Erfindung geeignete Derivate sind von yaad-Xa-dewA-his (SEQ ID NO: 20), yaad-Xa-rodA-his (SEQ ID NO: 22) oder yaad-Xa-basf1-his (SEQ ID NO: 24) durch Verkürzung des yaad-Fusionspartners abgeleitete Reste. Anstelle des vollständigen yaad-Fusionspartners (SEQ ID NO: 16) mit 294 Aminosäuren kann vorteilhaft ein verkürzter yaad-Rest eingesetzt werden. Der verkürzte Rest sollte aber zumindest 20, bevorzugt mindestens 35 Aminosäuren umfassen. Beispielsweise kann ein verkürzter Rest mit 20 bis 293, bevorzugt 25 bis 250, besonders bevorzugt 35 bis 150 und beispielsweise 35 bis 100 Aminosäuren eingesetzt werden.

Eine Spaltstelle zwischen dem Hydrophobin und dem Fusionspartner beziehungsweise den Fusionspartnern kann dazu genutzt, das reine Hydrophobin in underivatisierter Form freizusetzen (beispielsweise durch BrCN-Spaltung an Methionin, Faktor Xa-, Enterokinase-, Thrombin-, TEV-Spaltung etc.).

Es ist weiterhin möglich, Fusionsproteine aus einem Fusionspartner, beispielsweise yaad oder yaae, und mehreren Hydrophobinen, auch unterschiedlicher Sequenz, beispielsweise DewA-RodA oder Sc3-DewA, Sc3-RodA), hintereinander zu generieren. Ebenso können Hydrophobinfragmente (beispielsweise N- oder C-terminale Verkürzungen) oder Mutein, die bis zu 70% Homologie aufweisen, eingesetzt werden. Die Auswahl der optimalen Konstrukte erfolgt jeweils in Bezug auf die jeweilige Verwendung, d.h. die zu trennenden flüssigen Phasen.

Die erfindungsgemäß verwendeten Hydrophobine, beziehungsweise die in den erfindungsgemäßen Formulierungen enthaltenen Hydrophobine lassen sich chemisch durch bekannte Verfahren der Peptidsynthese, beispielsweise durch Festphasensynthese nach Merrifield herstellen.

Natürlich vorkommende Hydrophobine lassen sich aus natürlichen Quellen mittels geeigneter Methoden isolieren. Beispielhaft sei auf Wösten et. al., Eur. J Cell Bio. 63, 122-129 (1994) oder WO 96/41882 verwiesen.

Die Herstellung von Fusionsproteinen kann bevorzugt durch gentechnische Verfahren erfolgen, bei denen eine für den Fusionspartner und eine für den Hydrophobinteil codierende Nukleinsäuresequenz, insbesondere DNA-Sequenz, so kombiniert werden, dass in einem Wirtsorganismus durch Genexpression der kombinierten Nukleinsäuresequenz das gewünschte Protein erzeugt wird. Ein derartiges Herstellverfahren ist beispielsweise in DE 10 2005 007 480.4 offenbart.

Geeignete Wirtsorganismen (Produktionsorganismen) für das genannte Herstellverfahren können dabei Prokaryonten (einschließlich der Archaea) oder Eukaryonten sein, besonders Bakterien einschließlich Halobacterien und Methanococcen, Pilze, Insektenzellen, Pflanzenzellen und Säugerzellen, besonders bevorzugt Escherichia coli, Bacillus subtilis, Bacillus megaterium, Aspergillus oryzea, Aspergillus nidulans, Aspergillus niger, Pichia pastoris, Pseudomonas spec., Lactobacillen, Hansenula polymorpha, Trichoderma reesei, SF9 (bzw. verwandte Zellen) u.a..

Grundlage der Erfindung ist außerdem die Verwendung von Expressionskonstrukten, enthaltend unter der genetischen Kontrolle regulativer Nukleinsäuresequenzen, eine für ein erfindungsgemäß verwendetes Polypeptid kodierende Nukleinsäuresequenz, sowie Vektoren, umfassend wenigstens eines dieser Expressionskonstrukte. Vorzugsweise umfassen eingesetzte Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz. Unter einer "operativen Verknüpfung" wird im Rahmen der vorliegenden Erfindung die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann, verstanden.

Beispiele für operativ verknüpfbare Sequenzen sind Targeting-Sequenzen sowie Enhancer, Polyadenylierungssignale und dergleichen.

Weitere regulative Elemente umfassen selektierbare Marker, Amplifikationssignale, Replikationsursprünge und dergleichen. Geeignete regulatorische Sequenzen sind z. B. beschrieben in Goeddel, Gene Expression Technology : Methods in Enzymology 185, Academic Press, San Diego, CA (1990).

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde.

Ein bevorzugtes Nukleinsäurekonstrukt enthält vorteilhaft auch eine oder mehrere sogenannte "Enhancer"-Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren.

Die Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Vorteilhafte Regulationssequenzen für die Herstellung sind beispielsweise in Promotoren wie cos-, tac-, trp-, tet-, trp-tet-, lpp-, lac-, lpp-lac-, laclq-T7-, T5-, T3-, gal-, trc-, ara-, rhaP(rhaPBAD) SP6-, lambda-PR-oder imlambda-P-Promotor enthalten, die vorteilhaft in gram-negativen Bakterien Anwendung finden. Weitere vorteilhafte Regulationssequenzen sind beispielsweise in den gram-positiven Promotoren amy und SP02, in den Hefe- oder Pilzpromotoren ADC1, MFalpha, AC, P-60, CYC1, GAPDH, TEF, rp28, ADH enthalten.

Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z. B. Viren, wie SV40, CMV, Baculovirus und Adenovirus, Transposons,IS- Elemente, Phasmide, Cosmide, und lineare oder zirkuläre DNA, sowie das Agrobacterium-System zu verstehen.

Diese Vektoren können autonom im Wirtsorganismus repliziert oder chromosomal repliziert werden. Geeignete Plasmide sind beispielsweise in E. coli pLG338, pACYC184, pBR322, pUC18,pUC19, pKC30, pRep4, pHS1, pKK223-3, pDHE19.2, pHS2, pPLc236, pMBL24, pLG200, pUR290,pIN-III"3-B1, tgt11 oder pBdCl, in Streptomyces plJ101, plJ364, plJ702 oder plJ361, in Bacillus pUB110, pC194 oder pBD214, in Corynebacterium pSA77 oder pAJ667, in Pilzen pALS1, plL2 oder pBB116, in Hefen 2alpha, pAG-1, YEp6, YEp13 oder pEMBLYe23 oder in Pflanzen pLGV23,pGHlac+, pBIN19, pAK2004 oder pDH51. Die genannten Plasmide stellen eine kleine Auswahl der möglichen Plasmide dar. Weitere Plasmide sind dem Fachmann bekannt und können beispielsweise aus dem Buch Cloning Vectors (Eds. Pouwels P. H. et al. Elsevier, Amsterdam-New York-Oxford, 1985, ISBN 0 444 904018) entnommen werden.

Vorteilhaft enthält das Nukleinsäurekonstrukt zur Expression der weiteren enthaltenen Gene zusätzlich noch 3'-und/oder 5'-terminale regulatorische Sequenzen zur Steigerung der Expression, die je nach ausgewähltem Wirtorganismus und Gen oder Gene für eine optimale Expression ausgewählt werden.

Diese regulatorischen Sequenzen sollen die gezielte Expression der Gene und der Proteinexpression ermöglichen. Dies kann beispielsweise je nach Wirtsorganismus bedeuten, dass das Gen erst nach Induktion exprimiert oder überexprimiert wird, oder dass es sofort exprimiert und/oder überexprimiert wird.

Die regulatorischen Sequenzen bzw. Faktoren können dabei vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

In einer weiteren Ausgestaltungsform des Vektors kann der das Nukleinsäurekonstrukt oder die Nukleinsäure enthaltende Vektor auch vorteilhaft in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der Nukleinsäure bestehen.

Für eine optimale Expression heterologer Gene in Organismen ist es vorteilhaft die Nukleinsäuresequenzen entsprechend des im Organismus verwendeten spezifischen "codon usage" zu verändern. Der "codon usage" lässt sich anhand von Computerauswertungen anderer, bekannter Gene des betreffenden Organismus leicht ermitteln.

Die Herstellung einer Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E. F.Fritsch und J. Sambrook, Molecular Cloning : A Laboratory Manual, Cold Spring Harbor Laboratory, Cold Spring Harbor, NY (1989) sowie in T. J. Silhavy, M. L. Berman und L. W. Enquist, Experiments with Gene Fusions, Cold Spring Harbor Laboratory, Cold Spring Harbor, NY (1984) und in Ausubel, F. M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus, vorteilhaft in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

Mit Hilfe der Vektoren sind rekombinante Mikroorganismen herstellbar, welche beispielsweise mit wenigstens einem Vektor transformiert sind und zur Produktion der erfindungsgemäß verwendeten Hydrophobine oder Derivate davon eingesetzt werden können. Vorteilhafterweise werden die oben beschriebenen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning : A Laboratory Manual. 2. Aufl., Cold Spring Harbor Laboratory, Cold Spring Harbor Laboratory Press, Cold Spring Harbor, NY, 1989 beschrieben.

Es sind auch homolog rekombinierte Mikroorganismen herstellbar. Dazu wird ein Vektor hergestellt, der zumindest einen Abschnitt eines zu verwendenden Gens oder einer kodierenden Sequenz enthält, worin gegebenenfalls wenigstens eine Aminosäure-Deletion, -Addition oder -Substitution eingebracht worden ist, um die Sequenz zu verändern, z. B. funktionell zu disruptieren ("Knockout"- Vektor). Die eingebrachte Sequenz kann z. B. auch ein Homologes aus einem verwandten Mikroorganismus sein oder aus einer Säugetier-, Hefe- oder Insektenquelle abgeleitet sein. Der zur homologen Rekombination verwendete Vektor kann alternativ derart ausgestaltet sein, dass das endogene Gen bei homologer Rekombination mutiert oder anderweitig verändert ist, jedoch noch das funktionelle Protein kodiert (z. B. kann der stromaufwärts gelegene regulatorische Bereich derart verändert sein, dass dadurch die Expression des endogenen Proteins verändert wird). Der veränderte Abschnitt des erfindungsgemäß verwendeten Gens ist im homologen Rekombinationsvektor. Die Konstruktion geeigneter Vektoren zur homologen Rekombination ist z. B. beschrieben in Thomas, K. R. und Capecchi, M. R. (1987) Cell 51 : 503.

Als rekombinante Wirtsorganismen für derartige Nukleinsäuren oder derartige Nukleinsäurekonstrukte kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gramnegative Bakterien, bevorzugt Bakterien der Familien Enterobacteriaceae, Pseudomonadaceae, Rhizobiaceae, Streptomycetaceae oder Nocardiaceae, besonders bevorzugt Bakterien der Gattungen Escherichia, Pseudomonas, Streptomyces, Nocardia, Burkholderia, Salmonella, Agrobacterium oder Rhodococcus verwendet.

Die im oben beschriebenen Herstellverfahren für Fusionsproteine verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan- und Magnesiumsalze sowie gegebenenfalls Vitamine enthält, bei Temperaturen zwischen 0 und 100 °C, bevorzugt zwischen 10 bis 60 °C unter Sauerstoffbegasung angezogen. Dabei kann der pH-Wert der Nährflüssigkeit auf einem festen Wert gehalten werden, das heißt während der Anzucht reguliert werden oder nicht. Die Anzucht kann "batch"-weise, "semi-batch"-weise oder kontinuierlich erfolgen. Nährstoffe können zu Beginn der Fermentation vorgelegt oder semikontinuierlich oder kontinuierlich nachgefüttert werden. Die Enzyme können nach dem in den Beispielen beschriebenen Verfahren aus den Organismen isoliert werden oder als Rohextrakt für die Reaktion verwendet werden.

Die erfindungsgemäß verwendeten Hydrophobine oder funktionelle, biologisch aktive Fragmente davon können mittels eines Verfahrens zur rekombinanten Herstellung hergestellt werden, wobei man einen Polypeptide produzierenden Mikroorganismus kultiviert, gegebenenfalls die Expression der Proteine induziert und diese aus der Kultur isoliert. Die Proteine können so auch in großtechnischem Maßstab produziert werden, falls dies erwünscht ist. Der rekombinante Mikroorganismus kann nach bekannten Verfahren kultiviert und fermentiert werden. Bakterien können beispielsweise in TB- oder LB-Medium und bei einer Temperatur von 20 bis 40°C und einem pH-Wert von 6 bis 9 vermehrt werden. Im Einzelnen werden geeignete Kultivierungsbedingungen beispielsweise in T. Maniatis, E. F. Fritsch and J. Sambrook, Molecular Cloning : A Laboratory Manual, Cold Spring Harbor Laboratory, Cold Spring Harbor, NY (1989) beschrieben.

Die Zellen werden dann, falls die Proteine nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z. B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Proteine kann mit bekannten, chromatographischen Verfahren erzielt werden, wie Molekularsieb-Chromatographie (Gelfiltration), wie Q- Sepharose-Chromatographie, lonenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Water de Gruyter, Berlin, New York oder in Scopes, R., Protein Purification, Springer Verlag, New York, Heidelberg, Berlin beschrieben.

Besonders vorteilhaft kann es sein, die Fusions-Hydrophobine zur Erleichterung der Isolierung und Reinigung mit speziellen Ankergruppen zu versehen, die an entsprechende komplementäre Gruppen an festen Trägern, insbesondere geeigneten Polymeren anbinden können. Derartige feste Träger können beispielsweise als Füllung für Chromatographiesäulen verwendet werden, und auf diese Art und Weise kann die Effizienz der Trennung in der Regel deutlich gesteigert werden. Solche Trennverfahren sind auch als Affinitätschromatographie bekannt. Zum Einbau der Ankergruppen kann man bei der Herstellung der Proteine Vektorsysteme oder Oligonukleotide verwenden, die die cDNA um bestimmte Nukleotidsequenzen verlängern und damit veränderte Proteine oder Fusionsproteine kodieren. Zur leichteren Reinigung modifizierte Proteine umfassen als Anker fungierende sogenannte "Tags", wie beispielsweise die als Hexa-Histidin-Anker bekannte Modifikation. Mit Histidin-Ankern modifizierte Fusions-Hydrophobine lassen sich beispielsweise unter Verwendung von Nickel-Sepharose als Säulenfüllung chromatographisch reinigen. Das Fusions-Hydrophobin kann anschließend mittels geeigneten Mitteln zum Eluieren, wie beispielsweise einer Imidazol-Lösung, wieder von der Säule eluiert werden.

In einem vereinfachten Reinigungsverfahren kann auf die chromatographische Reinigung verzichtet werden. Hierzu werden die Zellen zunächst mittels einer geeigneten Methode aus der Fermetationsbrühe abgetrennt, beispielsweise durch Mikrofiltration oder durch Zentrifugieren. Anschließend können die Zellen mittels geeigneter Methoden, beispielsweise mittels der bereits oben genannten Methoden, aufgeschlossen, und die Zelltrümmer von den Einschlusskörpern (inclusion bodies) getrennt werden. Letzteres kann vorteilhaft durch Zentrifugieren erfolgen. Schließlich können die Einschlusskörper in prinzipiell bekannter Art und Weise aufgeschlossen werden, um die Fusions-Hydrophobine freizusetzen. Dies kann beispielsweise durch Säuren, Basen und/oder Detergentien erfolgen. Die Einschlusskörper mit den erfindungsgemäß verwendeten Fusion-Hydrophobinen können in der Regel schon unter Verwendung von 0,1 m NaOH innerhalb von ca. 1 h vollständig gelöst werden. Die Reinheit der nach diesem vereinfachten Verfahren erhaltenen Fusions-Hydrophobine liegt in der Regel bei 60 bis 80 Gew. % bzgl. der Menge aller Proteine. Die nach dem beschriebenen, vereinfachten Reinigungsverfahren erhaltenen Lösungen können ohne weitere Reinigung zur Ausführung dieser Erfindung eingesetzt werden.

Die wie beschrieben hergestellten Hydrophobine können sowohl direkt als Fusionsproteine als auch nach Abspaltung und Abtrennung des Fusionspartners als "reine" Hydrophobine verwendet werden.

Wenn eine Abtrennung des Fusionspartners vorgesehen ist, empfiehlt es sich eine potentielle Spaltstelle (spezifische Erkennungsstelle für Proteasen) in das Fusionsprotein zwischen Hydrophobinteil und Fusionspartnerteil einzubauen. Als Spaltstelle geeignet sind insbesondere solche Peptidsequenzen, die ansonsten weder im Hydrophobinteil noch im Fusionspartnerteil vorkommen, was sich mit bioinformatischen Tools leicht ermitteln lässt. Besonders geeignet sind beispielsweise BrCN-Spaltung an Methionin, oder durch Protease vermittelte Spaltung mit Faktor Xa-, Enterokinase-, Thrombin, TEV-Spaltung (Tobacca etch virus Protease).

Erfindungsgemäß können die Hydrophobine oder Derivate davon in Bohrspülungen enthaltend mindestens eine flüssige Phase eingesetzt werden. Dabei kann es sich um beliebige Zusammensetzungen handeln, solange diese mindestens eine flüssige Phase, insbesondere eine auf Öl basierende Phase, aufweisen. Die Zusammensetzung kann dabei im Rahmen der vorliegenden Erfindung auch weitere Phasen aufweisen.

Erfindungsgemäß handelt es sich bei Öl in Öl-basierten Bohrspülungen vorzugsweise um Biodiesel, ein internes Olefin, ein α-Olefin, einen pflanzlichen Ester, ein Paraffin oder eine Mischung daraus.

Geeignete weitere Lösungsmittel sind z. B. organische Lösungsmittel wie Ether, aromatische Verbindungen wie Toluol, Alkohole, Alkane, Alkene, Cycloalkane, Cycloalkene, Ester, Ketone, Naphtene oder halogenierte Kohlenwasserstoffe.

Die Bohrspülung kann auf die Art der vorzunehmenden Bohrung sowie auf gegebenenfalls im Erdreich vorhandene Stoffe (Erze, Salze, Bodenschätze) eingestellt werden, um eine optimale Wirkung zu erzielen. Das Bohren unter Einsatz der Bohrspülung kann auch durch eine erhöhte Temperatur zusätzlich unterstützt werden, beispielsweise eine Temperatur von 0 bis 400°C, beispielsweise von 30 bis 200 °C, insbesondere von 40 bis 150°C.

Die Menge des eingesetzten Hydrophobins oder Derivats davon kann in weiten Bereichen variieren, wobei die Menge vorteilhafterweise auf die Zusammensetzung an sich und gegebenenfalls in der Zusammensetzung enthaltene weitere Komponenten abgestimmt wird.

Die Bohrspülung (insbesondere deren Dichte) muss den jeweiligen Bedingungen wie z.B. Beschaffenheit der zu durchbohrenden Formation, der Bohrtiefe, dem Druck und der Temperatur angepasst werden.

Überraschenderweise wurde gefunden, dass bereits geringe Mengen eines Hydrophobins oder Derivats davon, zu einer Verbesserung der Stabilität der Bohrspülungen und damit auch des Bohrvorgangs führen.

Das mindestens eine Hydrophobin oder Derivat davon kann erfindungsgemäß in jeder geeigneten Menge eingesetzt werden. In der Regel wird das mindestens eine Hydrophobin oder Derivat davon in einer Menge von 0,1 bis 10.000 ppm, bezogen auf die gesamte Zusammensetzung, eingesetzt; bevorzugt in einer Menge von 1 bis 1000 ppm, besonders bevorzugt von 1 bis 600 ppm und ganz besonders bevorzugt 4 bis 500 ppm. Im Rahmen der vorliegenden Anmeldung bezeichnet die Angabe ppm mg pro kg.

Die erfindungsgemäßen Bohrspülungen können mit weiteren üblichen Komponenten und Additiven kombiniert werden. Hier sind beispielsweise Trägeröle ohne oder mit ausgeprägter Detergenswirkung zu nennen.

Geeignete mineralische Trägeröle sind bei der Erdölverarbeitung anfallende Fraktionen, wie Brightstock oder Grundöle mit Viskositäten wie beispielsweise aus der Klasse SN 500-2000; aber auch aromatische Kohlenwasserstoffe, paraffinische Kohlenwasserstoffe und Alkoxyalkanole. Erfindungsgemäß geeignet ist ebenfalls eine als "hydrocrack oil" bekannte und bei der Raffination von Mineralöl anfallende Fraktion (Vakuumdestillatschnitt mit einem Siedebereich von etwa 360 bis 500°C, erhältlich aus unter Hochdruck katalytisch hydriertem und isomerisiertem sowie entparaffiniertem natürlichen Mineralöl). Ebenfalls geeignet sind Mischungen oben genannter mineralischer Trägeröle.

Beispiele für erfindungsgemäß verwendbare synthetische Trägeröle sind ausgewählt unter: Polyolefinen (Polyalphaolefine oder Polyinternalolefine), (Poly)estern, (Poly)alkoxylaten, Polyethern, aliphatischen Polyetheraminen, alkylphenolgestarteten Polyethern, alkylphenolgestarteten Polyetheraminen und Carbonsäureester langkettiger Alkanole. Beispiele für geeignete Polyolefine sind Olefinpolymerisate mit Mn = 400 bis 1800, vor allem auf Polybuten- oder Polyisobuten-Basis (hydriert oder nicht hydriert).

Weitere geeignete Trägerölsysteme sind beispielsweise beschrieben in DE-A 38 26 608, DE-A 41 42 241, DE-A 43 09 074, EP-A 0 452 328 und EP-A 0 548 617, worauf hiermit ausdrücklich Bezug genommen wird. Die genannten Trägeröle werden dabei in dem Fachmann für die jeweilige Anwendung geeignet erscheinenden Mengen eingesetzt.

Weitere übliche Additive sind Korrosionsinhibitoren, beispielsweise auf Basis von zur Filmbildung neigenden Ammoniumsalzen organischer Carbonsäuren oder von heterocyclischen Aromaten im Falle von Buntmetallkorrosionsschutz; Antioxidantien oder Stabilisatoren, beispielsweise auf Basis von Aminen; weitere herkömmliche Emulgatoren; Antistatikmittel; Metallocene wie Ferrocen; Schmierfähigkeitsverbesserer (Lubricity-Additive) wie bestimmte Fettsäuren, Alkenylbernsteinsäureester, Bis(hydroxyalkyl)fettamine, Hydroxyacetamide oder Ricinusöl; Farbstoffe (Marker), Fluid Loss Additive (z.B. Polymere), Substanzen zum Einstellen der Dichte, Rheologie-Modifizierer, sowie Substanzen, die einen Filterkuchen aufbauen (wie z.B. Bentonit oder Clay), Kalk oder weitere Emulgatoren. Gegebenenfalls werden auch Amine zur Absenkung des pH-Wertes zugesetzt.

Die Erfindung wird im Folgenden durch Beispiele näher erläutert.

### Beispiele

### Beispiel 1

### Vorarbeiten für die Klonierung von yaad-His₆/ yaaE-His₆

Mit Hilfe der Oligonukleotide Hal570 und Hal571 (Hal 572/ Hal 573) wurde eine Polymerase Kettenreaktion durchgeführt. Als Template DNA wurde genomische DNA des Bakteriums Bacillus subtilis verwendet. Das erhaltene PCR Fragment enthielt die codierende Sequenz des Gens yaaD / yaaE aus Bacillus subtilis, und an den Enden je eine Ncol bzw. Bglll Restriktionsschnittstelle. Das PCR Fragment wurde gereinigt und mit den Restriktionsendonukleasen Ncol und Bglll geschnitten. Dieses DNA Fragment wurde als Insert verwendet, und in den zuvor mit den Restriktionsendonukleasen Ncol und Bglll linearisierten Vektor pQE60 der Firma Qiagen kloniert. Die so entstandenen Vektoren pQE60YAAD#2 / pQE60YaaE#5 können zur Expression von Proteinen bestehend aus, YAAD::HIS₆ bzw. YAAE::HIS₆ verwendet werden.
Ha1570: gcgcgcccatggctcaaacaggtactga
Ha1571: gcagatctccagccgcgttcttgcatac
Ha1572: ggccatgggattaacaataggtgtactagg
Ha1573: gcagatcttacaagtgccttttgcttatattcc

### Beispiel 2

### Klonierung von yaad-Hydrophobin DewA-His₆

Mit Hilfe der Oligonukleotide KaM 416 und KaM 417 wurde eine Polymerase Kettenreaktion durchgeführt. Als Template DNA wurde genomische DNA des Schimmelpilzes Aspergillus nidulans verwendet. Das erhaltene PCR Fragment enthielt die codierende Sequenz des Hydrophobin Gens dewA und einer N-Terminalen FaktorXa Proteinase Schnittstelle. Das PCR Fragment wurde gereinigt und mit der Restriktionsendonuklease BamHl geschnitten. Dieses DNA Fragment wurde als Insert verwendet, und in den zuvor mit der Restriktionsendonuklease Bglll linearisierten Vektor pQE60YAAD#2 kloniert.

Der so entstandene Vektor #508 kann zur Expression eines Fusionsproteins bestehend aus, YAAD::Xa::dewA::HIS₆, verwendet werden.
KaM416: GCAGCCCATCAGGGATCCCTCAGCCTTGGTACCAGCGC
KaM417: CCCGTAGCTAGTGGATCCATTGAAGGCCGCAT-GAAGTTCTCCGTCTCCGC

### Beispiel 3

### Klonierung von yaad-Hydrophobin RodA-His₆

Die Klonierung des Plasmids #513 erfolgte analog zu Plasmid #508 unter Verwendung der Oligonukleotide KaM 434 und KaM 435.
KaM434: GCTAAGCGGATCCATTGAAGGCCGCATGAAGTTCTCCATTGCTGC
KaM435: CCAATGGGGATCCGAGGATGGAGCCAAGGG

### Beispiel 4

### Klonierung von yaad-Hydrophobin BASF1-His₆

Die Klonierung des Plasmids #507 erfolgte analog zu Plasmid #508 unter Verwendung der Oligonukleotide KaM 417 und KaM 418.

Als Template DNA wurde ein künstlich synthetisierte DNA Sequenz - Hydrophobin BASF1 -eingesetzt (siehe Anhang, SEQ ID NO. 11 und 12).
KaM417:CCCGTAGCTAGTGGATCCATTGAAGGCCGCATGAAGTTCTCCGTCTCCG C
KaM418: CTGCCATTCAGGGGATCCCATATGGAGGAGGGAGACAG

### Beispiel 5

### Klonierung von yaad-Hydrophobin BASF2-His₆

Die Klonierung des Plasmids #506 erfolgte analog zu Plasmid #508 unter Verwendung der Oligonukleotide KaM 417 und KaM 418.

Als Template DNA wurde ein künstlich synthetisierte DNA Sequenz - Hydrophobin BASF2 -eingesetzt (siehe Anhang, SEQ ID NO. 13 und 14).
KaM417:CCCGTAGCTAGTGGATCCATTGAAGGCCGCATGAAGTTCTCCGTCTCCG C
KaM418: CTGCCATTCAGGGGATCCCATATGGAGGAGGGAGACAG

### Beispiel 6

### Klonierung von yaad-Hydrophobin SC3-His₆

Die Klonierung des Plasmids #526 erfolgte analog zu Plasmid #508 unter Verwendung der Oligonukleotide KaM464 und KaM465.

Als Template DNA wurde cDNA von Schyzophyllum commune eingesetzt (siehe Anhang, SEQ ID NO. 9 und 10).
KaM464: CGTTAAGGATCCGAGGATGTTGATGGGGGTGC
KaM465: GCTAACAGATCTATGTTCGCCCGTCTCCCCGTCGT

### Beispiel 7

### Fermentation des rekombinanten E.coli Stammes yaad-Hydrophobin DewA-His₆

Inokulation von 3ml LB Flüssigmedium mit einem yaad-Hydrophobin DewA-HiS₆ exprimierenden E.coli Stamm in 15ml Greiner Röhrchen. Inkubation für 8h bei 37°C auf einem Schüttler mit 200 UpM. Je 211 Erlenmeyer Kolben mit Schikanen und 250ml LB Medium (+ 100µg/ml Ampicillin) werden mit jeweils 1 ml der Vorkultur angeimpft und 9h bei 37°C auf einem Schüttler mit 180 UpM inkubiert.

13,51 LB-Medium (+100µg/ml Ampicillin) in einem 201 Fermenter mit 0,51 Vorkultur (OD₆₀₀ₙₘ 1:10 gegen H₂O gemessen) animpfen. Bei einer OD₆₀ₙₘ von -3.5 Zugabe von 140ml 100mM IPTG. Nach 3h Fermenter auf 10°C abkühlen und Fermentationsbrühe abzentrifugieren. Zellpellet zur weiteren Aufreinigung verwenden.

### Beispiel 8

### Reinigung des rekombinanten Hydrohobin-Fusionsproteins

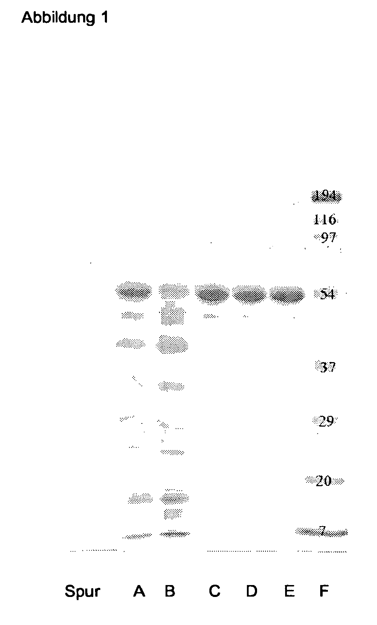

100 g Zellpellet (100 - 500 mg Hydrophobin) werden mit 50 mM Natriumphosphatpuffer, pH 7,5 auf 200 ml Gesamtvolumen aufgefüllt und resuspendiert. Die Suspension wird mit einem Ultraturrax Typ T25 (Janke und Kunkel; IKA-Labortechnik) für 10 Minuten behandelt und anschließend für 1 Stunde bei Raumtemperatur mit 500 Einheiten Benzonase (Merck, Darmstadt; Best.-Nr. 1.01697.0001) zum Abbau der Nukleinsäuren inkubiert. Vor dem Zellaufschluss wird mit einer Glaskartusche (P1) filtriert. Zum Zellaufschluß und für das Scheren der restlichen genomischen DNA werden zwei Homogenisatorläufe bei 1.500 bar durchgeführt (Microfluidizer M-110EH; Microfluidics Corp.). Das Homogenisat wird zentrifugiert (Sorvall RC-5B, GSA-Rotor, 250 ml Zentrifugenbecher, 60 Minuten, 4°C, 12.000 Upm, 23.000 g), der Überstand auf Eis gestellt und das Pellet in 100 ml Natriumphosphatpuffer, pH 7,5 resuspendiert. Zentrifugation und Resuspendieren werden dreimal wiederholt, wobei der Natriumphosphatpuffer bei der dritten Wiederholung 1 % SDS enthält. Nach der Resuspension wird für eine Stunde gerührt und eine abschließende Zentrifugation durchgeführt (Sorvall RC-5B, GSA-Rotor, 250 ml Zentrifugenbecher, 60 Minuten, 4°C, 12.000 Upm, 23.000 g). Gemäß SDS-PAGE Analyse ist das Hydrophobin nach der abschließenden Zentrifugation im Überstand enthalten (Abbildung 1). Die Versuche zeigen, dass das Hydrophobin wahrscheinlich in Form von Einschlusskörpern in den entsprechenden E.coli Zellen enthalten ist. 50 ml des Hydrophobin enthaltenden Überstandes werden auf eine 50 ml Nickel-Sepharose High Performance 17-5268-02 Säule aufgetragen (Amersham), die mit 50 mM Tris-Cl pH 8,0 Puffer äquilibriert wurde. Die Säule wird mit 50 mM Tris-Cl pH 8,0 Puffer gewaschen und das Hydrophobin anschließend mit 50 mM Tris-Cl pH 8,0 Puffer, der 200 mM Imidazol enthält, eluiert. Zur Entfernung des Imidazols wird die Lösung gegen 50 mM Tris-Cl pH 8,0 Puffer dialysiert.

### Abbildung 1 zeigt die Reinigung des hergestellten Hydrophobins:

| | |
|---|---|
| Spur A: | Auftrag Nickel-Sepharose Säule (1:10 Verdünnung) |
| Spur B: | Durchlauf = Eluat Waschschritt |
| Spuren C - E: | OD 280 Maxima der Elutionsfraktionen (WP1, WP2, WP3) |

Spur F zeigt den aufgetragenen Marker.

Das Hydrophobin der Abbildung 1 besitzt ein Molekulargewicht von ca. 53 kD. Die kleineren Banden repräsentieren zum Teil Abbauprodukte des Hydrophobins.

### Beispiel 9

### Anwendungstechnische Prüfung: Charakterisierung des Hydrophobins durch Kontaktwinkeländerung eines Wassertropfens auf Glas

### Substrat:

Glas (Fensterglas, Süddeutsche Glas, Mannheim):

Es wurde das gemäß Beispiel 8 gereinigte Hydrophobin eingesetzt.
- Konzentration des Hydrophobins in der Lösung: 100 µg/ml, die Lösung enthielt weiterhin 50 mM Na-Acetat-Puffer sowie 0,1 % Polyoxyethylen(20)-sorbitan-monolaureat (Tween^{®} 20)), pH-Wert der Lösung: 4
- Eintauchen von Glasplättchen in diese Lösung über Nacht (Temperatur 80°C)
- Danach wird das mit Hydrophobin beschichtete Glasplättchen der Lösung entnommen und in destilliertem Wasser gewaschen,
- Danach Inkubation 10min / 80°C /1% SDS-Lösung in destilliertem Wasser
- Erneutes Waschen in destilliertem Wasser

Die Proben werden an der Luft getrocknet und der Kontaktwinkel (in Grad) eines Tropfens von 5 µl Wasser mit der beschichteten Glasoberfläche bei Raumtemperatur bestimmt.

Die Kontaktwinkelmessung wurde auf einem Gerät Dataphysics Contact Angle System OCA 15+, Software SCA 20.2.0. (November 2002) bestimmt. Die Messung erfolgte gemäß den Herstellerangaben.

Unbehandeltes Glas ergab einen Kontaktwinkel von 30 ± 5°;

Das mit dem Hydrophobin gemäß Beispiel 8 (yaad-dewA-his₆) beschichtete Glasplättchen ergab einen Kontaktwinkel von 75 ± 5°. ==> Zunahme des Kontaktwinkels: 45°

### Beispiel 10

### Herstellung einer Bohrspülung auf Öl-Basis enthaltend ein Hydrophobin-Konzentrat (yaad-Xa-dewA-His₆)

Das Hydrophobin-Konzentrat wurde in einer Menge von
a) 0 ppm
b) 10 ppm
c) 100 ppm
d) 10.000 ppm
einer Formulierung enthaltend 222 ml Biodiesel sowie 61 ml einer Kalziumchlorid-Lösung (25 %ig) zugesetzt.

Mit einem Emulgierungsgerät (Ultra Turrax T50) wurden in verschiedenen Testreihen bei den Drehzahlen 2000, 6000 und 10000 U/min sowie den Rührzeiten 5 und 10 Minuten verschiedene Emulsionen hergestellt.

Mit den Bohrspülungs-Proben wurden jeweils Emulsionstests (analog DIN 51415) durchgeführt.

Hierbei werden die Komponenten miteinander gemischt, wobei die Bedingungen (Rührzeit und Drehgeschwindigkeit) angegeben werden. Danach wird der Entmischungsvorgang in Abhängigkeit der Zeit beobachtet.

Die Auswertung geschieht anhand von Standards, wobei
1 für eine vollständige Spaltung der Emulsion,
2 für eine teilweise Spaltung unter Freisetzung von Wasser,
3 eine nicht feststellbare Spaltung der Emulsion stehen.

In Tabelle 1 sind Ergebnisse der Versuche für die genanten Hydrophobin-Konzentrationen zusammengestellt. Aufgeführt sind jeweils die Beurteilungen der Phasentrennschichten nach 1 Minute, 24 Stunden und 48 Stunden (jeweils bei Raumtemperatur, da 23 ° C). Ferner werden auch Versuche bei einer gleich bleibenden Temperatur von 80° C durchgeführt.

**Tabelle 1**

| Beispiel | Bedingungen | 1 Min | 24 h | 48 h. | 24h, 80°C |
|---|---|---|---|---|---|
| 10 a (Vergleich) | 5 min. 6000 U/min. | 3 | 1 | 1 | 1 |
| 10 a (Vergleich) | 10 min. 6000U/min | 3 | 1 | 1 | 1 |
| 10 b | 5 min. 6000 U/min. | 3 | 3 | 3 | 1 |
| 10 b | 10 min. 6000U/min | 3 | 3 | 3 | 1 |
| 10 c | 5 min. 6000 U/min. | 3 | 3 | 3 | 2 |
| 10 c | 10 min. 6000U/min | 3 | 3 | 3 | 2 |
| 10 d | 5 min. 6000 U/min. | 3 | 3 | 3 | 2 |
| 10 d | 10 min. 6000U/min | 3 | 3 | 3 | 3 |

### Kommentar:

Ohne Zusatz von Hydrophobin A wird eine beschleunigte Aufspaltung der Bohrmischung beobachtet. Die Bewertung 1 ist für die Praxis der Ölbohrung nicht akzeptabel. Die Hydrophobine weisen schon in äußerst geringen Mengen einen guten emulgierenden Effekt auf. Schon 10 ppm Hydrophobin (weniger als 1 mg) führen zu einem akzeptablen Ergebnis. Bei der Verwendung von Hydrophobin in höherer Dosierung werden noch bessere Ergebnisse erzielt, insbesondere hinsichtlich der Thermostabilität.

### Zuordnung der Sequenznamen zu DNA- und Polypeptidsequenzen im Sequenzprotokoll

| | |
|---|---|
| dewA DNA- und Polypeptidsequenz | SEQ ID NO: 1 |
| dewA Polypeptidsequenz | SEQ ID NO: 2 |
| rodA DNA- und Polypeptidsequenz | SEQ ID NO: 3 |
| rodA Polypeptidsequenz | SEQ ID NO: 4 |
| hypA DNA- und Polypeptidsequenz | SEQ ID NO: 5 |
| hypA Polypeptidsequenz | SEQ ID NO: 6 |
| hypB DNA- und Polypeptidsequenz | SEQ ID NO: 7 |
| hypB Polypeptidsequenz | SEQ ID NO: 8 |
| sc3 DNA- und Polypeptidsequenz | SEQ ID NO: 9 |
| sc3 Polypeptidsequenz | SEQ ID NO: 10 |
| basf1 DNA- und Polypeptidsequenz | SEQ ID NO: 11 |
| basf1 Polypeptidsequenz | SEQ ID NO: 12 |
| basf2 DNA- und Polypeptidsequenz | SEQ ID NO: 13 |
| basf2 Polypeptidsequenz | SEQ ID NO: 14 |
| yaad DNA- und Polypeptidsequenz | SEQ ID NO: 15 |
| yaad Polypeptidsequenz | SEQ ID NO: 16 |
| yaae DNA- und Polypeptidsequenz | SEQ ID NO: 17 |
| yaae Polypeptidsequenz | SEQ ID NO: 18 |
| yaad-Xa-dewA-his DNA- und Polypeptidsequenz | SEQ ID NO: 19 |
| yaad-Xa-dewA-his Polypeptidsequenz | SEQ ID NO: 20 |
| yaad-Xa-rodA-his DNA- und Polypeptidsequenz | SEQ ID NO: 21 |
| yaad-Xa-rodA-his Polypeptidsequenz | SEQ ID NO: 22 |
| yaad-Xa-basf1-his DNA- und Polypeptidsequenz | SEQ ID NO: 23 |
| yaad-Xa-basf1-his Polypeptidsequenz | SEQ ID NO: 24 |

## Patentansprüche

1. Verwendung eines Hydrophobins als Hilfsmittel in einer Bohrspülung.

2. Verwendung nach Anspruch 1, **dadurch gekennzeichnet, dass** das Hydrophobin als Emulgator in einer Bohrspülung eingesetzt wird.

3. Verwendung nach einem der Ansprüche 1 oder 2, **dadurch gekennzeichnet, dass** das eingesetzte Hydrophobin ein Fusions-Hydrophobin ist.

4. Verwendung nach einem der Ansprüche 1 bis 3, **dadurch gekennzeichnet, dass** das eingesetzte Hydrophobin ein Fusions-Hydrophobin ausgewählt aus der Gruppe von yaad-Xa-dewA-his, yaad-Xa-rodA-his oder yaad-Xa-basf1-his ist, wobei es sich bei yaad auch um einen verkürzten Fusionspartner yaad' mit 20 bis 293 Aminosäuren handeln kann.

5. Verwendung nach einem der Ansprüche 1 bis 4, **dadurch gekennzeichnet, dass** das Hydrophobin als Hilfsmittel für eine Bohrspülung auf ÖI-Basis dient.

6. Verwendung nach einem der Ansprüche 1 bis 5, **dadurch gekennzeichnet, dass** das Hydrophobin in einer Menge von 0,1 bis 10.000. ppm, bezogen auf die gesamte Zusammensetzung, eingesetzt wird.

7. Verwendung nach einem der Ansprüche 1 bis 6, **dadurch gekennzeichnet, dass** das Hydrophobin in einer Menge von 1 bis 1.000 ppm, bezogen auf die gesamte Zusammensetzung, eingesetzt wird.

8. Verwendung nach einem der Ansprüche 1 bis 7, **dadurch gekennzeichnet, dass** es sich um eine Bohrspülung auf ÖI-Basis handelt, die 40 bis 95 Gew.-% mindestens einer Ölkomponente, 2 bis 60 Gew.-% Wasser, sowie gegebenenfalls weitere Komponenten enthält.

9. Verwendung nach einem der Ansprüche 1 bis 8, **dadurch gekennzeichnet, dass** neben dem Hydrophobin mindestens eine weitere Verbindung eingesetzt wird, die die Emulsionsbildung verbessert.

10. Verfahren zur Bohrung eines Bohrloches zur Erschließung von unterirdischen Lagerstätten, bei dem eine Bohrspülung eingesetzt wird, die mindestens ein Hydrophobin enthält.

11. Verfahren nach Anspruch 10, **dadurch gekennzeichnet, dass** das in der Bohrspülung eingesetzte Hydrophobin ein Fusions-Hydrophobin oder ein Derivat davon ist.

12. Verfahren nach einem der Ansprüche 10 oder 11, **dadurch gekennzeichnet, dass** das in der Bohrspülung eingesetzte Hydrophobin ein Fusions-Hydrophobin ausgewählt aus der Gruppe von yaad-Xa-dewA-his, yaad-Xa-rodA-his oder yaad-Xa-basf1-his ist, wobei es sich bei yaad auch um einen verkürzten Fusionspartner yaad' mit 20 bis 293 Aminosäuren handeln kann.

13. Verfahren nach einem der Ansprüche 10 bis 12, **dadurch gekennzeichnet, dass** das Hydrophobin als Hilfsmittel für eine Bohrspülung auf Öl-Basis dient.

14. Verfahren nach einem der Ansprüche 10 bis 13, **dadurch gekennzeichnet, dass** das Hydrophobin in einer Menge von 0,1 bis 10.000 ppm, bezogen auf die gesamte Zusammensetzung, eingesetzt wird.

15. Verfahren nach einem der Ansprüche 10 bis 14, **dadurch gekennzeichnet, dass** das Hydrophobin in einer Menge von 1 bis 1.000 ppm, bezogen auf die gesamte Zusammensetzung, eingesetzt wird.

16. Verfahren nach einem der Ansprüche 10 bis 15, **dadurch gekennzeichnet, dass** es sich um eine Bohrspülung auf Öl-Basis handelt, die 40 bis 95 Gew.-% mindestens einer Ölkomponente, 2 bis 60 Gew.-% Wasser, sowie gegebenenfalls weitere Komponenten enthält.

17. Verfahren nach einem der Ansprüche 10 bis 16, **dadurch gekennzeichnet, dass** es sich um eine Bohrspülung auf Öl-Basis handelt, die 70 bis 95 Gew.-% mindestens einer Ölkomponente, 2 bis 30 Gew.-% Wasser, sowie gegebenenfalls weitere Komponenten enthält.

18. Verfahren nach einem der Ansprüche 10 bis 17, **dadurch gekennzeichnet, dass** es sich um eine Bohrspülung auf ÖI-Basis handelt, die 70 bis 95 Gew.-% mindestens einer Ölkomponente, 2 bis 30 Gew.-% Wasser, sowie gegebenenfalls bis zu 13 Gew.-% weiterer Komponenten enthält.

19. Verfahren nach einem der Ansprüche 10 bis 18, **dadurch gekennzeichnet, dass** neben dem Hydrophobin mindestens eine weitere Verbindung eingesetzt wird, die die Emulsionsbildung verbessert.

20. Bohrspülung enthaltend mindestens ein Hydrophobin.

21. Bohrspülung nach Anspruch 20, **dadurch gekennzeichnet, dass** das Hydrophobin ein Fusions-Hydrophobin oder ein Derivat davon ist.

22. Bohrspülung nach einem der Ansprüche 20 oder 21, **dadurch gekennzeichnet, dass** das in der Bohrspülung eingesetzte Hydrophobin ein Fusions-Hydrophobin ausgewählt aus der Gruppe von yaad-Xa-dewA-his, yaad-Xa-rodA-his oder yaad-Xa-basf1-his ist, wobei es sich bei yaad auch um einen verkürzten Fusionspartner yaad' mit 20 bis 293 Aminosäuren handeln kann.

23. Bohrspülung nach einem der Ansprüche 20 bis 22, **dadurch gekennzeichnet, dass** es sich um eine Bohrspülung auf Öl-Basis handelt.

24. Bohrspülung nach einem der Ansprüche 20 bis 23, **dadurch gekennzeichnet, dass** sie das Hydrophobin in einer Menge von 0,1 bis 10.000 ppm, bezogen auf die gesamte Zusammensetzung, enthält.

25. Bohrspülung nach einem der Ansprüche 20 bis 24, **dadurch gekennzeichnet, dass** sie das Hydrophobin in einer Menge von 1 bis 1.000 ppm, bezogen auf die gesamte Zusammensetzung, enthält.

26. Bohrspülung nach einem der Ansprüche 20 bis 25, **dadurch gekennzeichnet, dass** es sich um eine Bohrspülung auf Öl-Basis handelt, die 40 bis 95 Gewichtsprozent mindestens einer Ölkomponente, 2 bis 60 Gewichtsprozent Wasser, sowie gegebenenfalls weitere Komponenten enthält.

27. Bohrspülung nach einem der Ansprüche 20 bis 26, **dadurch gekennzeichnet, dass** es sich um eine Bohrspülung auf Öl-Basis handelt, die 70 bis 95 Gewichtsprozent mindestens einer Ölkomponente, 2 bis 30 Gewichtsprozent Wasser, sowie gegebenenfalls weitere Komponenten enthält.

28. Bohrspülung nach einem der Ansprüche 20 bis 27, **dadurch gekennzeichnet, dass** es sich um eine Bohrspülung auf Öl-Basis handelt, die 70 bis 95 Gewichtsprozent mindestens einer Ölkomponente, 2 bis 30 Gewichtsprozent Wasser, sowie gegebenenfalls bis zu 13 Gewichtsprozent weiterer Komponenten enthält.

29. Bohrspülung nach einem der Ansprüche 20 bis 28, **dadurch gekennzeichnet, dass** sie neben mindestens einem Hydrophobin mindestens eine weitere Verbindung enthält, die die Emulsionsbildung verbessert.

30. Verfahren zur Herstellung einer Bohrspülung nach einem der Ansprüche 20 bis 29, **dadurch gekennzeichnet, dass** die Hydrophobin-Komponente mit den übrigen Komponenten der Bohrspülung innig vermischt wird.

## Claims

1. The use of a hydrophobin as an assistant in a drilling mud.

2. The use according to claim 1, wherein the hydrophobin is used as an emulsifier in a drilling mud.

3. The use according to either of claims 1 and 2, wherein the hydrophobin used is a fusion hydrophobin.

4. The use according to any of claims 1 to 3, wherein the hydrophobin used is a fusion hydrophobin selected from the group of yaad-xa-dewA-his, yaad-Xa-rodA-his or yaad-Xa-basf1-his, where yaad may also be a truncated fusion partner yaad' having from 20 to 293 amino acids.

5. The use according to any of claims 1 to 4, wherein the hydrophobin serves as an assistant for an oil-based drilling mud.

6. The use according to any of claims 1 to 5, wherein the hydrophobin is used in an amount of from 0.1 to 10 000 ppm, based on the overall composition.

7. The use according to any of claims 1 to 6, wherein the hydrophobin is used in an amount of from 1 to 1000 ppm, based on the overall composition.

8. The use according to any of claims 1 to 7, wherein the drilling mud is an oil-based drilling mud which comprises from 40 to 95% by weight of at least one oil component, from 2 to 60% by weight of water and if appropriate further components.

9. The use according to any of claims 1 to 8, wherein at least one further compound which improves the emulsion formation is used as well as the hydrophobin.

10. A process for drilling a borehole for developing underground deposits, in which a drilling mud which comprises at least one hydrophobin is used.

11. The process according to claim 10, wherein the hydrophobin used in the drilling mud is a fusion hydrophobin or a derivative thereof.

12. The process according to either of claims 10 and 11, wherein the hydrophobin used in the drilling mud is a fusion hydrophobin selected from the group of yaad-Xa-dewA-his, yaad-Xa-rodA-his or yaad-Xa-basf1-his, where yaad may also be a truncated fusion partner yaad' having from 20 to 293 amino acids.

13. The process according to any of claims 10 to 12, wherein the hydrophobin serves as an assistant for an oil-based drilling mud.

14. The process according to any of claims 10 to 13, wherein the hydrophobin is used in an amount of from 0.1 to 10 000 ppm, based on the overall composition.

15. The process according to any of claims 10 to 14, wherein the hydrophobin is used in an amount of from 1 to 1000 ppm, based on the overall composition.

16. The process according to any of claims 10 to 15, wherein the drilling mud is an oil-based drilling mud which comprises from 40 to 95% by weight of at least one oil component, from 2 to 60% by weight of water and if appropriate further components.

17. The process according to any of claims 10 to 16, wherein the drilling mud is an oil-based drilling mud which comprises from 70 to 95% by weight of at least one oil component, from 2 to 30% by weight of water and if appropriate further components.

18. The process according to any of claims 10 to 17, wherein the drilling mud is an oil-based drilling mud which comprises from 70 to 95% by weight of at least one oil component, from 2 to 30% by weight of water and if appropriate up to 13% by weight of further components.

19. The process according to any of claims 10 to 18, wherein at least one further compound which improves the emulsion formation is used as well as the hydrophobin.

20. A drilling mud comprising at least one hydrophobin.

21. The drilling mud according to claim 20, wherein the hydrophobin is a fusion hydrophobin or a derivative thereof.

22. The drilling mud according to either of claims 20 and 21, wherein the hydrophobin used in the drilling mud is a fusion hydrophobin selected from the group of yaad-Xa-dewA-his, yaad-Xa-rodA-his or yaad-Xa-basf1-his, where yaad may also be a truncated fusion partner yaad' having from 20 to 293 amino acids.

23. The drilling mud according to any of claims 20 to 22, which is an oil-based drilling mud.

24. The drilling mud according to any of claims 20 to 23, which comprises the hydrophobin in an amount of from 0.1 to 10 000 ppm, based on the overall composition.

25. The drilling mud according to any of claims 20 to 24, which comprises the hydrophobin in an amount of from 1 to 1000 ppm, based on the overall composition.

26. The drilling mud according to any of claims 20 to 25, which is an oil-based drilling mud which comprises from 40 to 95 percent by weight of at least one oil component, from 2 to 60 percent by weight of water and if appropriate further components.

27. The drilling mud according to any of claims 20 to 26, which is an oil-based drilling mud which comprises from 70 to 95 percent by weight of at least one oil component, from 2 to 30 percent by weight of water and if appropriate further components.

28. The drilling mud according to any of claims 20 to 27, which is an oil-based drilling mud which comprises from 70 to 95 percent by weight of at least one oil component, from 2 to 30 percent by weight of water and if appropriate up to 13 percent by weight of further components.

29. The drilling mud according to any of claims 20 to 28, which comprises, as well as at least one hydrophobin, at least one further compound which improves emulsion formation.

30. A process for producing a drilling mud according to any of claims 20 to 29, wherein the hydrophobin component is mixed intimately with the remaining components of the drilling mud.

## Revendications

1. Utilisation d'une hydrophobine comme adjuvant dans une boue de forage.

2. Utilisation selon la revendication 1, **caractérisée en ce que** l'hydrophobine est utilisée comme émulsifiant dans une boue de forage.

3. Utilisation selon l'une quelconque des revendications 1 ou 2, **caractérisée en ce que** l'hydrophobine utilisée est une hydrophobine de fusion.

4. Utilisation selon l'une quelconque des revendications 1 à 3, **caractérisée en ce que** l'hydrophobine utilisée est une hydrophobine de fusion choisie dans le groupe formé par yaad-Xa-dewA-his, yaad-Xa-rodA-his ou yaad-Xa-basf1-his, où yaad peut également être un partenaire de fusion plus court yaad' comprenant 20 à 293 acides aminés.

5. Utilisation selon l'une quelconque des revendications 1 à 4, **caractérisée en ce que** l'hydrophobine sert d'adjuvant pour une boue de forage à base d'huile.

6. Utilisation selon l'une quelconque des revendications 1 à 5, **caractérisée en ce que** l'hydrophobine est utilisée en une quantité de 0,1 à 10 000 ppm, par rapport à la composition totale.

7. Utilisation selon l'une quelconque des revendications 1 à 6, **caractérisée en ce que** l'hydrophobine est utilisée en une quantité de 1 à 1000 ppm, par rapport à la composition totale.

8. Utilisation selon l'une quelconque des revendications 1 à 7, **caractérisée en ce qu'**il s'agit d'une boue de forage à base d'huile, qui contient 40 à 95% en poids d'au moins un composant huileux, 2 à 60% en poids d'eau, ainsi que le cas échéant d'autres composants.

9. Utilisation selon l'une quelconque des revendications 1 à 8, **caractérisée en ce qu'**on utilise, outre l'hydrophobine, au moins un autre composé, qui améliore la formation d'une émulsion.

10. Procédé pour le forage d'un trou de forage pour l'exploitation de gisements souterrains, lors duquel on utilise une boue de forage qui contient au moins une hydrophobine.

11. Procédé selon la revendication 10, **caractérisé en ce que** l'hydrophobine utilisée dans la boue de forage est une hydrophobine de fusion ou un dérivé de celle-ci.

12. Procédé selon l'une quelconque des revendications 10 ou 11, **caractérisé en ce que** l'hydrophobine utilisée dans la boue de forage est une hydrophobine de fusion choisie dans le groupe formé par yaad-Xa-dewA-his, yaad-Xa-rodA-his ou yaad-Xa-basf1-his, où yaad peut également être un partenaire de fusion plus court yaad' comprenant 20 à 293 acides aminés.

13. Procédé selon l'une quelconque des revendications 10 à 12, **caractérisé en ce que** l'hydrophobine sert d'adjuvant pour une boue de forage à base d'huile.

14. Procédé selon l'une quelconque des revendications 10 à 13, **caractérisé en ce que** l'hydrophobine est utilisée en une quantité de 0,1 à 10 000 ppm, par rapport à la composition totale.

15. Procédé selon l'une quelconque des revendications 10 à 14, **caractérisé en ce que** l'hydrophobine est utilisée en une quantité de 1 à 1000 ppm, par rapport à la composition totale.

16. Procédé selon l'une quelconque des revendications 10 à 15, **caractérisé en ce qu'**il s'agit d'une boue de forage à base d'huile, qui contient 40 à 95% en poids d'au moins un composant huileux, 2 à 60% en poids d'eau, ainsi que le cas échéant d'autres composants.

17. Procédé selon l'une quelconque des revendications 10 à 16, **caractérisé en ce qu'**il s'agit d'une boue de forage à base d'huile, qui contient 70 à 95% en poids d'au moins un composant huileux, 2 à 30% en poids d'eau, ainsi que le cas échéant d'autres composants.

18. Procédé selon l'une quelconque des revendications 10 à 17, **caractérisé en ce qu'**il s'agit d'une boue de forage à base d'huile, qui contient 70 à 95% en poids d'au moins un composant huileux, 2 à 30% en poids d'eau, ainsi que le cas échéant jusqu'à 13% en poids d'autres composants.

19. Procédé selon l'une quelconque des revendications 10 à 18, **caractérisé en ce qu'**on utilise, outre l'hydrophobine, au moins un autre composé, qui améliore la formation d'une émulsion.

20. Boue de forage contenant au moins une hydrophobine.

21. Boue de forage selon la revendication 20, **caractérisée en ce que** l'hydrophobine est une hydrophobine de fusion ou un dérivé de celle-ci.

22. Boue de forage selon l'une quelconque des revendications 20 ou 21, **caractérisée en ce que** l'hydrophobine utilisée dans la boue de forage est une hydrophobine de fusion choisie dans le groupe formé par yaad-Xa-dewA-his, yaad-Xa-rodA-his ou yaad-Xa-basf1-his, où yaad peut également être un partenaire de fusion plus court yaad' comprenant 20 à 293 acides aminés.

23. Boue de forage selon l'une quelconque des revendications 20 à 22, **caractérisée en ce qu'**il s'agit d'une boue de forage à base d'huile.

24. Boue de forage selon l'une quelconque des revendications 20 à 23, **caractérisée en ce qu'**elle contient l'hydrophobine en une quantité de 0,1 à 10 000 ppm, par rapport à la composition totale.

25. Boue de forage selon l'une quelconque des revendications 20 à 24, **caractérisée en ce qu'**elle contient l'hydrophobine en une quantité de 1 à 1000 ppm, par rapport à la composition totale.

26. Boue de forage selon l'une quelconque des revendications 20 à 25, **caractérisée en ce qu'**il s'agit d'une boue de forage à base d'huile, qui contient 40 à 95% en poids d'au moins un composant huileux, 2 à 60% en poids d'eau, ainsi que le cas échéant d'autres composants.

27. Boue de forage selon l'une quelconque des revendications 20 à 26, **caractérisée en ce qu'**il s'agit d'une boue de forage à base d'huile, qui contient 70 à 95% en poids d'au moins un composant huileux, 2 à 30% en poids d'eau, ainsi que le cas échéant d'autres composants.

28. Boue de forage selon l'une quelconque des revendications 20 à 27, **caractérisée en ce qu'**il s'agit d'une boue de forage à base d'huile, qui contient 70 à 95% en poids d'au moins un composant huileux, 2 à 30% en poids d'eau, ainsi que le cas échéant jusqu'à 13% en poids d'autres composants.

29. Boue de forage selon l'une quelconque des revendications 20 à 28, **caractérisée en ce, qu'**elle contient, outre au moins une hydrophobine, au moins un autre composé, qui améliore la formation d'une émulsion.

30. Procédé pour la préparation d'une boue de forage selon l'une quelconque des revendications 20 à 29, **caractérisé en ce que** le composant hydrophobine est mélangé intimement avec les autres composants de la boue de forage.
